# EUROPEAN PATENT SPECIFICATION

(11) **EP 3 132 756 B1**
(45) Date of publication and mention of the grant of the patent: **11.11.2020**
(21) Application number: 16184652.2
(22) Date of filing: 18.08.2016
(51) Int. Cl.: A61B 17/128, A61B 17/29, A61B 90/00

(54) **SURGICAL CLIP APPLIER**
APPLIKATOR FÜR CHIRURGISCHE KLAMMERN
APPLICATEUR D'AGRAFES CHIRURGICALES

(30) Priority: 19.08.2015 US 201514829727
(43) Date of publication of application: 22.02.2017
(73) Proprietor: Covidien LP, Mansfield, MA 02048 (US)
(72) Inventor: ROCKROHR, Brian, Waterbury, CT 06705 (US); MALKOWSKI, Jaroslaw T., Trumbull, CT 06611 (US); CRESTON, Brian, West Haven, CT 06516 (US); ZAMMATARO, Thomas, Hamden, CT 06517 (US); MEEHAN, Christopher M, West Haven, CT 06516 (US); ADDI, Ernest, Middletown, CT 06457 (US)
(74) Representative: Soames, Candida Jane

(56) References cited:
- US-A1- 2014 194 903

## Description

### TECHNICAL FIELD

The present application relates to surgical instruments, and more particularly, to surgical clip appliers having a plurality of clips for applying the clips to body tissues and vessels during surgical procedures.

### BACKGROUND

Surgical clip appliers are known in the art and have increased in popularity among surgeons by offering an alternative to conventional suturing of body tissues and vessels. Typical instruments are disclosed in U.S. Pat. No. 5,030,226 to Green et al. and U.S. Pat. No. 5,431,668 to Burbank, III et al. These instruments generally provide a plurality of clips which are stored in the instrument and which are fed sequentially to the jaw mechanism at the distal end of the instrument upon opening and closing of the handles at the proximal end of the instrument. As the handles are closed, the jaws close to deform a clip positioned between the jaw members, and as the jaws are opened to release the deformed clip, a new clip is fed from the series to a position between the jaws. This process is repeated until all the clips in the series of clips have been used.

Inevitably, these instruments tend to be relatively complex, including many parts, many of which move and cooperate with one another.

Thus, in light of the increasing cost of healthcare and surgery, a need exists to curb the costs of the devices used during a surgical procedure or the like.

Accordingly, a need exists for an improved clip applier having fewer parts and which is more economical to manufacture.

### SUMMARY

The present application relates to surgical clip appliers having a plurality of clips for applying the clips to body tissues and vessels during surgical procedures and their methods of use.

A prior art surgical clip applier is disclosed in U.S. Patent Application 2014/0194903.

According to an aspect of the present disclosure, a surgical clip applier is provided and includes a housing; at least one handle pivotably connected to the housing; a channel assembly extending distally from the housing; a clip carrier disposed within the channel assembly and defining a channel; a plurality of clips loaded in the channel of the clip carrier; a drive channel translatably supported in the housing and the channel assembly, the drive channel being translated upon actuation of the at least one handle; and a ratchet mechanism disposed within the housing. The ratchet mechanism includes a rack member connected to the drive channel such that axial translation of the drive channel results in axial translation of the rack member, the rack member defining a first set of teeth along a first side thereof and a second set of teeth along a second side thereof; a first pawl and a second pawl each tiltably supported in the housing and disposed on respective opposed sides of the rack member; and a buckling spring interposed between the first pawl and the second pawl and constrained in a slot formed in the rack member, wherein the buckling spring is dimensioned so as to bow in one of a proximal direction and a distal direction.

In use, when the buckling spring is bowed in the proximal direction, the first pawl and the second pawl are engaged with the rack member so as to permit the drive channel to move in a distal direction; and, when the buckling spring is bowed in the distal direction, the first pawl and the second pawl are engaged with the rack member so as to permit the drive channel to move in a proximal direction.

The surgical clip applier may further include a clip follower slidably disposed within the channel of the clip carrier and disposed proximally of the plurality of clips, the clip follower being configured and adapted for selective incremental advancement through the channel of the clip carrier and through the channel assembly, wherein the clip follower is configured and adapted to urge the plurality of clips, in a distal direction relative to the clip carrier, following a loading of a distal-most clip of the plurality of clips into the pair of jaws.

The clip follower may be urged in a distal direction by a biasing member. The biasing member may be a constant force spring. The constant force spring may include a distal end secured against movement in the surgical clip applier, and a proximal end coiled onto itself. The coiled proximal end of the constant force spring may be connected to the clip follower so as to draw the clip follower distally upon a coiling of the coiled proximal end of the constant force spring.

The surgical clip applier may further include a clip pusher bar reciprocally positioned within at least one of the housing and the channel assembly. The pusher bar may have a first end operatively connected to the at least one handle and a second end defining a pusher. The pusher bar may be movable away from the pair of jaws as the at least one handle is actuated in order to move the pusher behind a distal-most clip stored in the channel of the clip carrier. The pusher bar may be configured and adapted to move towards the jaws as the at least one handle is returned to an un-actuated position to move the distal-most clip between the pair of jaws.

The clip follower may include a head configured and dimensioned for engagement by the pusher of the clip pusher bar, when in a retracted position, following a loading of a final clip of the stack of clips into a pair of jaws of the surgical clip applier.

In use, following engagement of the head of the clip follower by the pusher of the clip pusher bar, a distal advancement of the clip pusher bar may advance the clip follower distally such that the head of the clip follower is positioned between the pair of jaws.

In use, when the head of the clip follower is positioned between the pair of jaws, the head of the clip follower may prevent the pair of jaws from closing and thus prevents the at least one handle from actuating completely.

The surgical clip applier may further include a jaw assembly including a pair of jaws extending from an end of the channel assembly, opposite the housing, the jaw assembly adapted to accommodate a clip therein and being operable to effect formation of a clip in response to movement of the at least one handle.

The drive channel may include a first end operatively connected to the at least one handle and a second end configured and dimensioned to surround and selectively engage the pair of jaws to effectuate closure of the pair of jaws. The drive channel may be moved towards the jaw assembly as the at least one handle is moved in a first direction to move the second end of the drive channel against the pair of jaws to close the pair of jaws. The drive channel may be moved away from the jaw assembly as the at least one handle is moved in a second direction, opposite the first direction, to move the second end of the drive channel away from the jaw assembly to allow the pair of jaws to open.

The second end of the drive channel may include a tongue extending between the pair of jaws.

In use, with the drive channel disposed at a proximal-most position, with the buckling spring bowed in the proximal direction, and with the first pawl and the second pawl engaged with the rack member so as to permit the drive channel to move in a distal direction, when the drive channel is advanced to a distal-most position, the proximally bowing buckling spring may be acted on and caused to bow in the distal direction, whereby the drive channel is permitted to move in a proximal direction.

In use, with the drive channel disposed at a distal-most position, with the buckling spring bowed in the distal direction, and with the first pawl and the second pawl engaged with the rack member so as to permit the drive channel to move in a proximal direction, when the drive channel is retracted to the proximal-most position, the distally bowing buckling spring may be acted on and caused to bow in the proximal direction, whereby the drive channel is permitted to move in a distal direction.

The drive channel may include a stop tab projecting therefrom in a direction toward the clip follower, and wherein the clip follower defines a window therein. In use, when the clip follower is in a distal-most position with the head thereof disposed between the pair of jaws, and when the drive channel is in a proximal-most position, the stop tab of the drive channel may be disposed in the window of the clip follower.

The clip follower may include an elongate body having a distal end and a proximal end, wherein the head is supported at the distal end thereof; and a tail having a distal end and a proximal end, wherein the proximal end of the tail is connected to the proximal end of the elongate body such that the tail and the elongate body bias away from one another.

The window of the clip follower that configured to receive the stop tab of the drive channel may be formed in the tail.

The clip follower may be urged in a distal direction by a constant force spring. The constant force spring may include a distal end secured against movement in the surgical clip applier, and a proximal end coiled onto itself and at least partially disposed in the window of the tail of the clip follower. The coiled proximal end of the constant force spring may draw the clip follower distally upon a coiling of the coiled proximal end of the constant force spring subsequent to a loading of a distal-most clip of the plurality of clips into a pair of jaws of the surgical clip applier.

In use, when the buckling spring is caused to be buckled so as to bow from the proximal direction to the distal direction and from the distal direction to the proximal direction, the pair of pawls may be caused to flip about a pivot point and create at least one of an audible and tactile feedback.

The channel assembly may include a pair of opposed proximal side walls, wherein each proximal side wall of the channel assembly may define a substantially V-shaped channel, and wherein each pawl may be pivotably disposed in a respective V-shaped channel of the channel assembly.

Each V-shaped channel may include a rib projecting into the channel. The rib of the V-shaped channel may be received in a notch defined in a respective pawl, wherein an axial position of each pawl in the V-shaped notch is maintained.

The drive channel may include a tail extending proximally from a proximal end thereof. The tail of the drive channel may extend between the first pawl and the second pawl, and wherein the rack member of the ratchet mechanism may be formed on the tail of the drive channel.

According to yet another aspect of the present disclosure, a surgical clip applier includes a housing, one or more handles movably connected to the housing, and a channel assembly that extends from the housing. The surgical clip applier includes a clip carrier disposed within the channel assembly and defining a channel, a plurality of clips loaded in the channel of the clip carrier, and a drive channel movably supported in the housing and the channel assembly. A clip pusher bar is reciprocally positioned within one or both of the housing and the channel assembly. A linkage mechanism couples the drive channel to the clip pusher bar. The linkage mechanism has a driver link arm pivotally connected to a driven link arm. The surgical clip applier also includes one or more stop members operably associated with the linkage mechanism and configured to prevent premature lockout of the surgical clip applier.

The stop members may extend from the driver link arm, the driven link arm, a rack member associated with the drive channel, or combinations thereof. In some embodiments, one or more stop members selectively contacts the driver link arm, the driven link arm, or combinations thereof to limit movement of the driver link arm, the driven link arm, or combinations thereof.

### BRIEF DESCRIPTION OF THE DRAWINGS

The present clip applier will be more fully appreciated as the same becomes better understood from the following detailed description when considered in connection with the following drawings, in which:
FIG. 1 is a perspective view of a surgical clip applier according to an embodiment of the present disclosure;
FIG. 2 is a top, plan view of the surgical clip applier of FIG. 1;
FIG. 3 is side, elevational view of the surgical clip applier of FIGS. 1 and 2;
FIG. 4 is an exploded perspective view of the surgical clip applier of FIGS. 1-3;
FIG. 5 is a perspective view of a clip channel of the surgical clip applier of FIGS. 1-3;
FIG. 6 is an enlarged perspective view of a distal end of a pusher bar of the surgical clip applier of FIGS. 1-3;
FIG. 7 is a perspective view of a clip follower of the surgical clip applier of FIGS. 1-3;
FIG. 8 is an enlarged view of the indicated area of detail of FIG. 7;
FIG. 9 is an enlarged view of a proximal end of the clip follower illustrating a biasing member associated therewith;
FIG. 10 is a perspective view of a distal end of the surgical clip applier of FIGS. 1-3;
FIG. 11 is a perspective view, with parts separated of a pair of jaws and a distal end of a drive channel of the surgical clip applier of FIGS. 1-3;
FIG. 12 is a perspective view of the surgical clip applier of FIGS. 1-3 with a housing half-section removed therefrom;
FIG. 13 is a partial enlarged view of the surgical clip applier illustrated in FIG. 12;
FIG. 14 is an enlarged view of the indicated area of detail of FIG. 13;
FIG. 15 is a perspective view of the distal end of the surgical clip applier of FIGS. 1-3;
FIG. 16 is a perspective view of the distal end of the surgical clip applier of FIGS. 1-3 with the clip channel removed therefrom;
FIG. 17 is an enlarged view of the indicated area of detail of FIG. 13;
FIG. 18 is an enlarged view of the indicated area of detail of FIG. 13;
FIG. 19 is the partial enlarged view of the surgical clip applier illustrated in FIG. 13 with a stack of clips and a linkage mechanism removed;
FIG. 20 is an enlarged view of the indicated area of detail of FIG. 19;
FIG. 21 is the partial enlarged view of the surgical clip applier illustrated in FIG. 13 with the stack of clips, the linkage mechanism, and a pusher bar removed;
FIG. 22 is an enlarged view of the indicated area of detail of FIG. 21;
FIG. 23 is a perspective view of the surgical clip applier of FIGS. 1-3 with a housing half-section removed therefrom and illustrating the surgical clip applier in an un-actuated condition;
FIG. 24 is an enlarged view of the indicated area of detail of FIG. 23;
FIG. 25 is an enlarged view of the indicated area of detail of FIG. 23;
FIG. 26 is an enlarged view of the indicated area of detail of FIG. 24;
FIG. 27 is a longitudinal cross-sectional view of the surgical clip applier illustrated in FIG. 23;
FIG. 28 is an enlarged view of the indicated area of detail of FIG. 27;
FIG. 29 is an enlarged view of the indicated area of detail of FIG. 27;
FIG. 30 is an enlarged view of the indicated area of detail of FIG. 29;
FIG. 31 is an enlarged view of the indicated area of detail of FIG. 27;
FIG. 32 is a plan view of the surgical clip applier of FIGS. 1-3 with the housing removed therefrom and illustrating the surgical clip applier in an un-actuated condition;
FIG. 33 is an enlarged view of the indicated area of detail of FIG. 32;
FIG. 34 is a top, plan view of the pair of jaws and the drive channel of the surgical clip applier illustrated in FIG. 32;
FIG. 35 is a cross-sectional view as taken through 35-35 of FIG. 34;
FIG. 36 is an enlarged view of the indicated area of detail of FIG. 32;
FIG. 37 is a perspective view of the surgical clip applier of FIGS. 1-3 with the housing removed therefrom and illustrating the surgical clip applier during an initial actuation thereof;
FIG. 38 is an enlarged view of the indicated area of detail of FIG. 37;
FIG. 39 is an enlarged view of the indicated area of detail of FIG. 37;
FIG. 40 is a longitudinal cross-sectional view of the surgical clip applier illustrated in FIG. 37;
FIG. 41 is an enlarged view of the indicated area of detail of FIG. 40;
FIG. 42 is a top, plan view of the pair of jaws and the drive channel of the surgical clip applier illustrated in FIG. 37;
FIG. 43 is a cross-sectional view as taken through 43-43 of FIG. 40;
FIG. 44 is a top, plan view of the surgical clip applier of FIGS. 1-3 with the housing removed therefrom and illustrating the surgical clip applier in a fully actuated condition;
FIG. 45 is an enlarged view of the indicated area of detail of FIG. 44;
FIG. 46 is a top, plan view of the linkage mechanism and a ratchet mechanism of the surgical clip applier of FIG. 44 when the surgical clip applier in a fully actuated condition;
FIG. 47 is a top, plan view of the pair of jaws and the drive channel of the surgical clip applier of FIG. 44 when the surgical clip applier in a fully actuated condition;
FIG. 48 is a bottom, perspective view of the pair of jaws and the drive channel of the surgical clip applier of FIG. 47;
FIG. 49 is a top, perspective view of the pair of jaws, the drive channel, the clip channel and the stack of clips of the surgical clip applier of FIGS. 1-3 when the surgical clip applier in the fully actuated condition;
FIG. 50 is a top, perspective view of the pair of jaws, the drive channel and the clip channel of the surgical clip applier of FIGS. 1-3 when the surgical clip applier in the fully actuated condition;
FIG. 51 is a longitudinal, cross-sectional view of the distal end of the surgical clip applier illustrated in FIG. 44;
FIG. 52 is a perspective view of a body vessel including a clip of the surgical clip applier, shown applied thereto;
FIG. 53 is a top, plan view of the linkage mechanism illustrated during an opening of the surgical clip applier of FIGS. 1-3;
FIG. 54 is a longitudinal, cross-sectional view of the distal end of the surgical clip applier of FIGS. 1-3, illustrated during an opening of the surgical clip applier;
FIG. 55 is a longitudinal, cross-sectional view of the channel assembly of the surgical clip applier of FIGS. 1-3, illustrated in a locked out condition;
FIG. 56 is an enlarged view of the indicated area of detail of FIG. 55;
FIG. 57 is a longitudinal, cross-sectional view of the channel assembly of the surgical clip applier of FIGS. 1-3, illustrating a locking out of the surgical clip applier;
FIG. 58 is an enlarged view of the indicated area of detail of FIG. 55;
FIG. 59 is a perspective view of a rear end of a handle assembly of a surgical clip applier according to another embodiment of the present disclosure, with a housing half removed therefrom, illustrating a ratchet mechanism according to another embodiment of the present disclosure;
FIG. 60 is a top, plan view of the ratchet mechanism illustrated in FIG. 59;
FIG. 61 is a perspective view, with parts separated, of a lower channel, a drive channel, and the ratchet mechanism of the surgical clip applier illustrated in FIGS. 59 and 60;
FIG. 62 is an enlarged view of the indicated area of detail of FIG. 61;
FIG. 63 is a top, perspective view of a surgical clip applier, with a cover and a housing half-section removed therefrom, illustrating a clip follower according to another embodiment of the present disclosure;
FIG. 64 is an enlarged view of the indicated area of detail of FIG. 63;
FIG. 65 is a perspective view, with parts separated, illustrating the clip follower of FIG. 63 and a constant force spring according to another embodiment of the present disclosure;
FIG. 66 is an enlarged, perspective view illustrating a connection of the constant force spring of FIG. 63-66 to the cartridge cover;
FIG. 67 is a rear, bottom perspective view of the clip follower of FIGS. 63-65;
FIG. 68 is a front, top perspective view of the clip follower of FIGS. 63-65;
FIG. 69 is a perspective, longitudinal cross-sectional view of the clip applier illustrated in FIGS. 63-66;
FIG. 70 is an elevational, longitudinal cross-sectional view of the clip applier illustrated in FIGS. 63-66;
FIG. 71 is a side, perspective view of one embodiment of a driver link;
FIG. 72 is a side view of a portion of one embodiment of a clip applier with the drive link of FIG. 71 shown coupled thereto;
FIG. 73 is a perspective view of one embodiment of a driven link;
FIG. 74 is a side, perspective view of a portion of another embodiment of a clip applier with the driven link of FIG. 73 shown coupled thereto;
FIG. 75 is a side, perspective view of another embodiment of a driven link;
FIG. 76 is a side, perspective view of a portion of yet another embodiment of a clip applier with the driven link of FIG. 75 shown coupled thereto;
FIG. 77 is a perspective view of one embodiment of a ratchet plate; and
FIG. 78 is a side, perspective view of a portion of still another embodiment of a clip applier with the ratchet plate of FIG. 77 shown coupled thereto.

### DETAILED DESCRIPTION OF EMBODIMENTS

Embodiments of surgical clip appliers in accordance with the present disclosure will now be described in detail with reference to the drawing figures wherein like reference numerals identify similar or identical structural elements. As shown in the drawings and described throughout the following description, as is traditional when referring to relative positioning on a surgical instrument, the term "proximal" refers to the end of the apparatus which is closer to the user and the term "distal" refers to the end of the apparatus which is further away from the user.

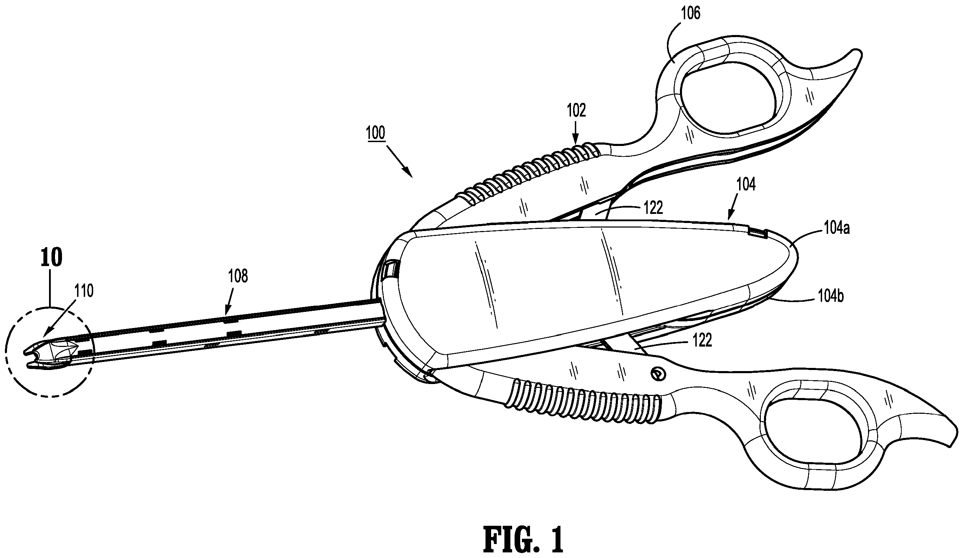

Turning initially to FIGS. 1-3, a surgical instrument that is capable of applying a number of hemostatic clips to selected tissue for occlusion purposes during open surgical procedures is generally designated as 100.

Clip applier 100 includes a handle assembly 102 including a housing 104 having an upper housing half 104a and lower housing half 104b. Handle assembly 102 further includes a pair of handles 106 pivotably secured to housing 104 and extending outwardly therefrom. A channel assembly 108 is fixedly secured to housing 104 and extends outwardly therefrom, terminating in and supporting a jaw assembly 110.

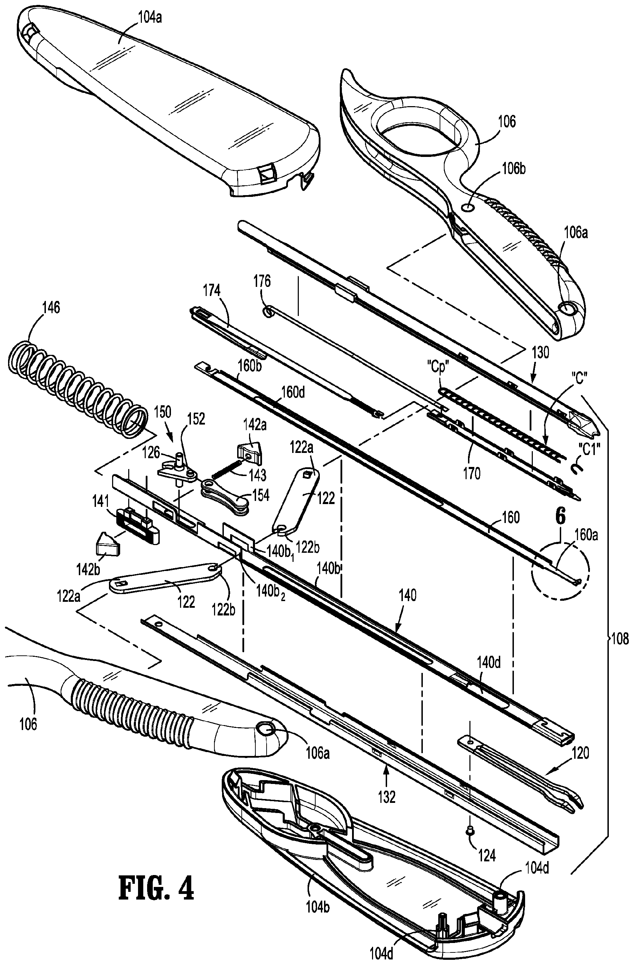

As seen in FIG. 4, housing halves 104a and 104b of clip applier 100 fit together by snap fit engagement with one another. Housing 104 is formed of a suitable plastic material.

Handles 106 are secured to housing 104 by complementary, two-part handle pivot posts 104d extending from upper housing half 104a and lower housing half 104b which complementary, two-part posts 104d couple with one another when housing halves 104a, 104b are connected or snapped to one another. Pivot posts 104d are received into respective distal apertures 106a formed in handles 106. Handle assembly 102 includes a link member 122 pivotally connected at a first end 122a thereof to each handle 106 at a pivot point 106b formed in a respective handle 106. A second end 122b of each link member 122 is pivotally connected to a respective pivot point 140b₁, 140b₂ defined in side walls 140b of drive channel 140.

Channel assembly 108 includes a channel or cartridge cover 130 and an outer or lower channel 132 each having a proximal end retained in housing assembly 102, between upper and lower housing halves 104a, 104b. Cartridge cover 130 includes at least one retention element configured and adapted to selectively engage, in a snap-fit engagement, a complementary or corresponding retention element provided on outer channel 132.

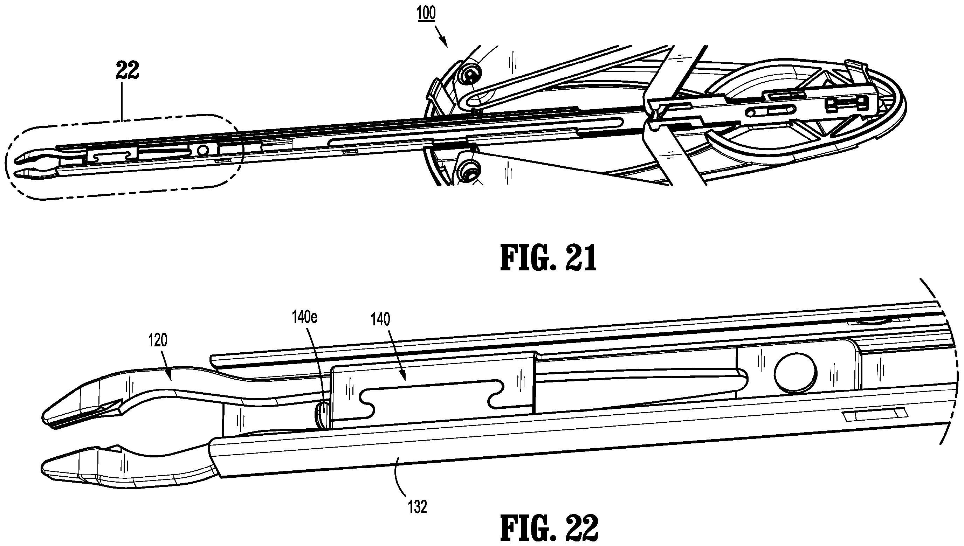

As seen in FIG. 4 and FIGS. 11, 21 and 22, clip applier 100 includes a drive channel 140 reciprocally supported in and extending between housing 104 of handle assembly 102 and channel assembly 108. A proximal end of a drive channel 140 is supported between upper and lower housing halves 104a, 104b of housing 104 and a distal end of drive channel 140 is supported between cartridge cover 130 and outer channel 132 of channel assembly 108.

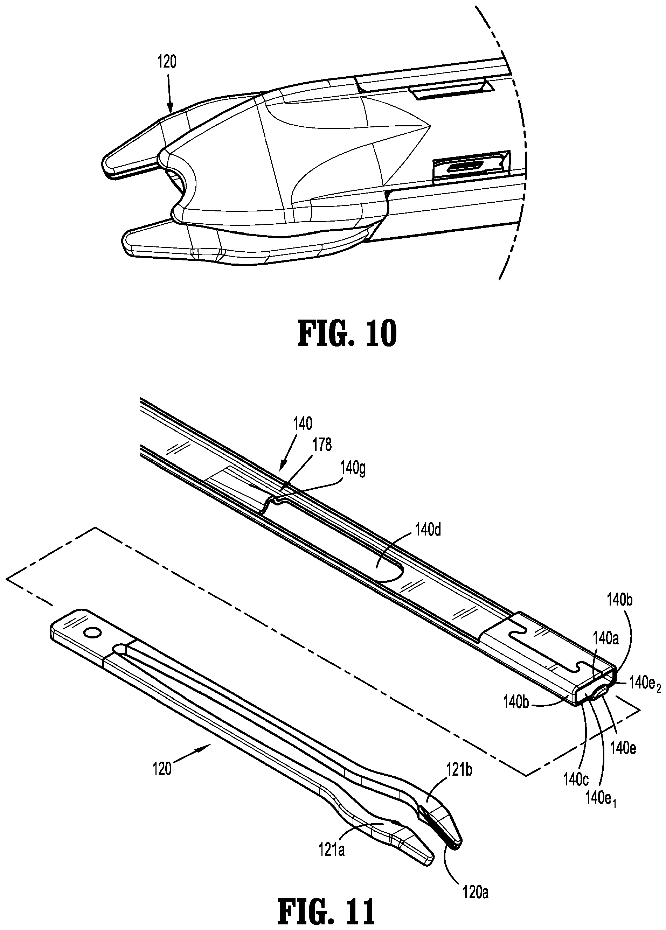

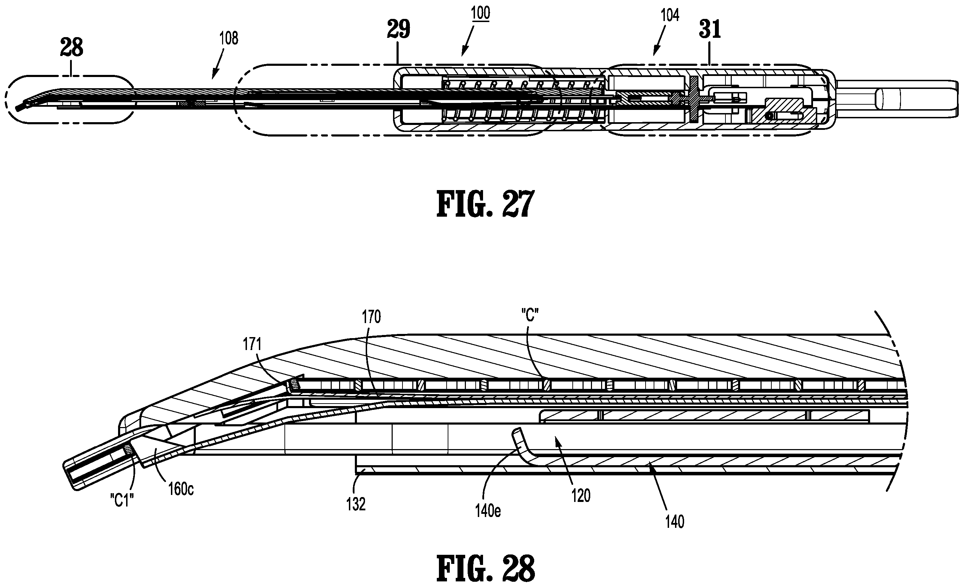

As seen in FIGS. 11 and 26-28, a distal end of drive channel 140 is a substantially box-shaped tube including a pair of spaced apart side walls 140b interconnecting a top wall 140a and a bottom wall 140c. The distal-most end of drive channel 140 includes a tongue 140e disposed between side walls 140b and extending from and between top wall 140a and bottom wall 140c to define a pair of distal-facing openings 140e₁, 140e₂.

A proximal end of drive channel 140 defines an elongate pin slot 140d formed in bottom wall 140c thereof for slidable passage of a pin 124 therealong. Pin 124 is located along a central, longitudinal axis of channel assembly 108.

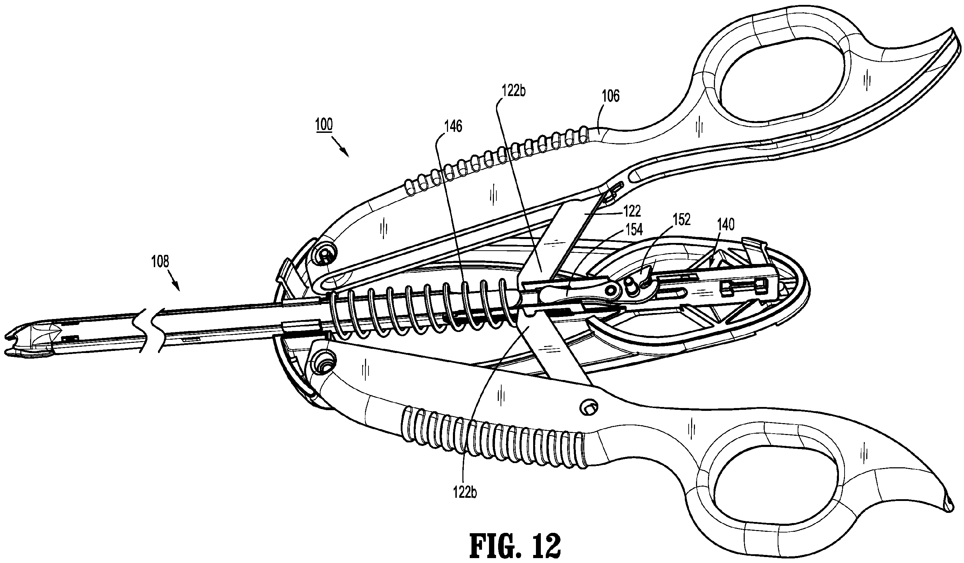

As seen in FIG. 4 and FIG. 12, clip applier 100 further includes a biasing member 146, in the form of a compression spring, disposed within housing 104 so as to operatively surround an intermediate portion of drive channel 140 and to abut against an inner distal surface of housing 104. Biasing member 146 functions to generally maintain drive channel 140 in a retracted or proximal-most position. In particular, biasing member 146 functions to retract or facilitate retraction of drive channel 140 following formation of a clip "C" positioned between jaws 120, and following release of handles 106.

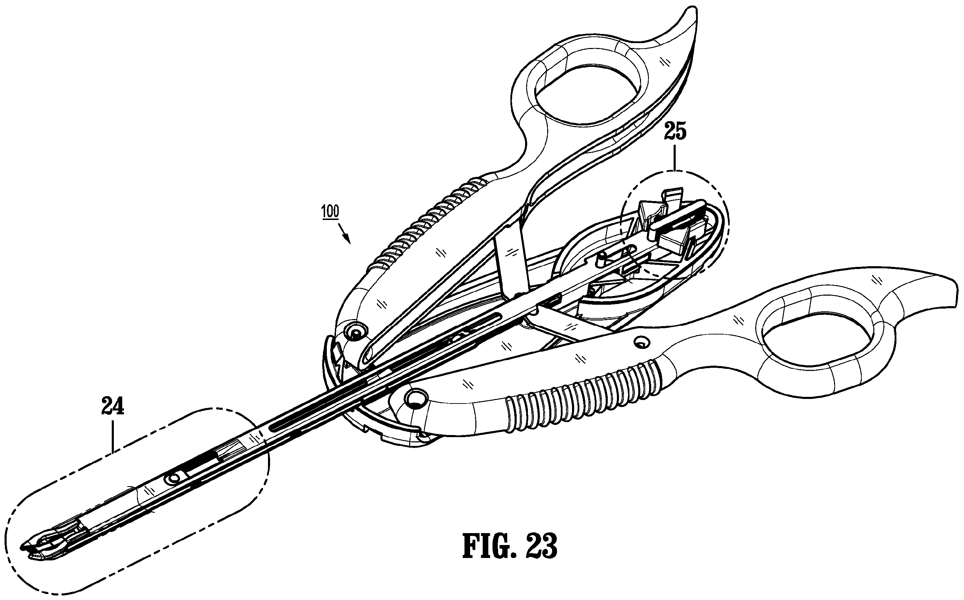

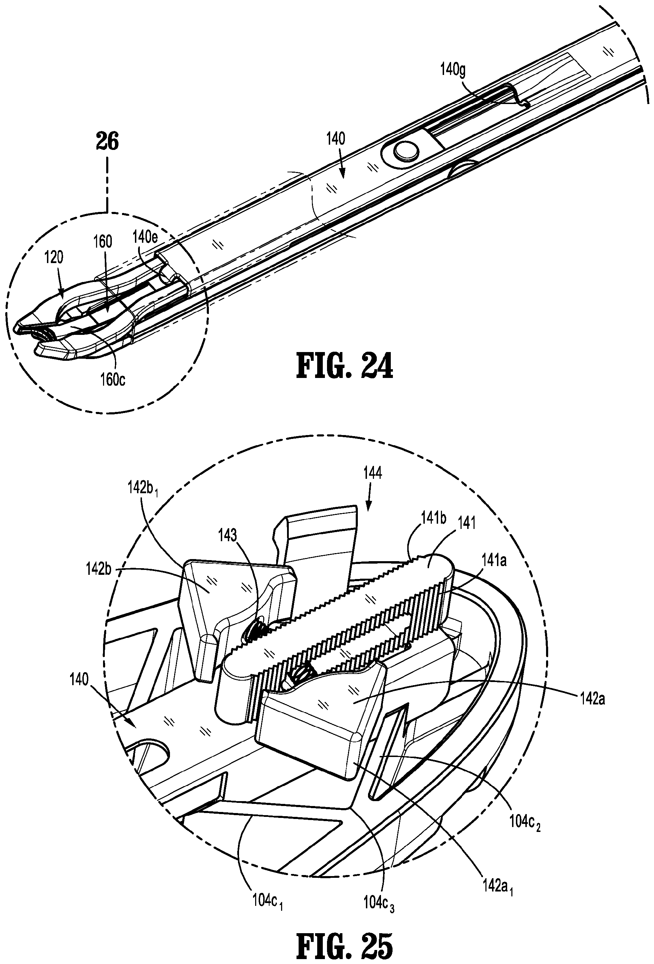

As seen in FIGS. 23 and 25, a proximal end of drive channel 140 supports a ratchet rack member 141 and secured thereto, wherein ratchet rack member 141 is movable together with drive channel 140. Ratchet rack member 141 defines a first set of teeth 141a configured and adapted to engage with a first ratchet pawl 142a, and a second set of teeth 141b configured and adapted to engage a second ratchet pawl 142b, wherein the first set and the second set of teeth 141a, 141b are disposed on opposed sides of rack member 141. First ratchet pawl 142a and second ratchet pawl 142b are each supported in housing 104, with first ratchet pawl 142a and second ratchet pawl 142b being disposed on opposed sides of ratchet rack member 141. Rack member 141 and pawls 142a, 142b define a ratchet mechanism 144. In use, as drive channel 140 is moved axially, rack member 141 is also moved axially therewith. In operation, pawls 142a, 142b reverse their direction when a distal/proximal end of a longitudinally extending slot formed in rack member 141 impacts a snap-over ratchet spring 143, causing ratchet spring 143 to flip, and in turn, causing pawls 142a, 142b to flip or reverse their direction, as will be discussed in greater detail below.

Each pawl 142a, 142b is pivotally supported in upper housing half 104a and lower housing half 104b at a location wherein each pawl 142a, 142b is in substantial operative engagement with respective rack teeth 141a, 141b of rack member 141. Pawls 142a, 142b are engageable with rack member 141 to restrict longitudinal movement of rack member 141 and, in turn, drive channel 140.

Additionally, as seen in FIG. 25, each pawl 142a, 142b has a substantially triangular profile, wherein a remote corner 142a₁, 142b₁ of each pawl 142a, 142b extends away from rack member 141 and is situated within an internal corner 104c₃ of a pair of walls 104c₁, 104c₂ defined in upper housing half 104a and lower housing half 104b. The interface of the remote corner 142a₁, 142b₁ of each pawl 142a, 142b and internal corner 104c₃ of a pair of walls 104c₁, 104c₂ defines a pivot point for pawls 142a, 142b.

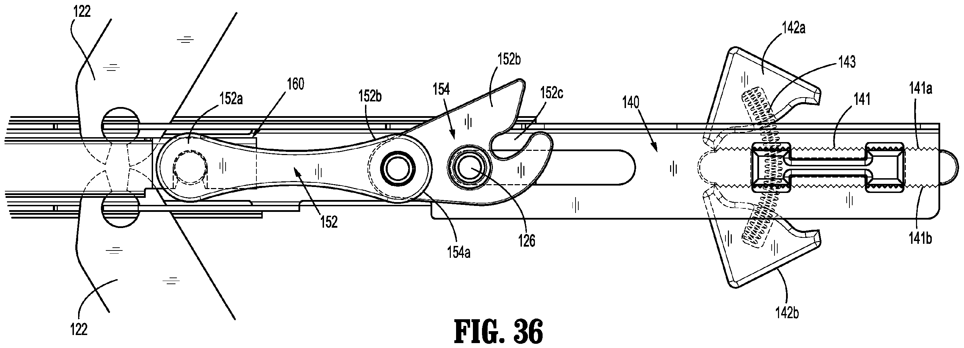

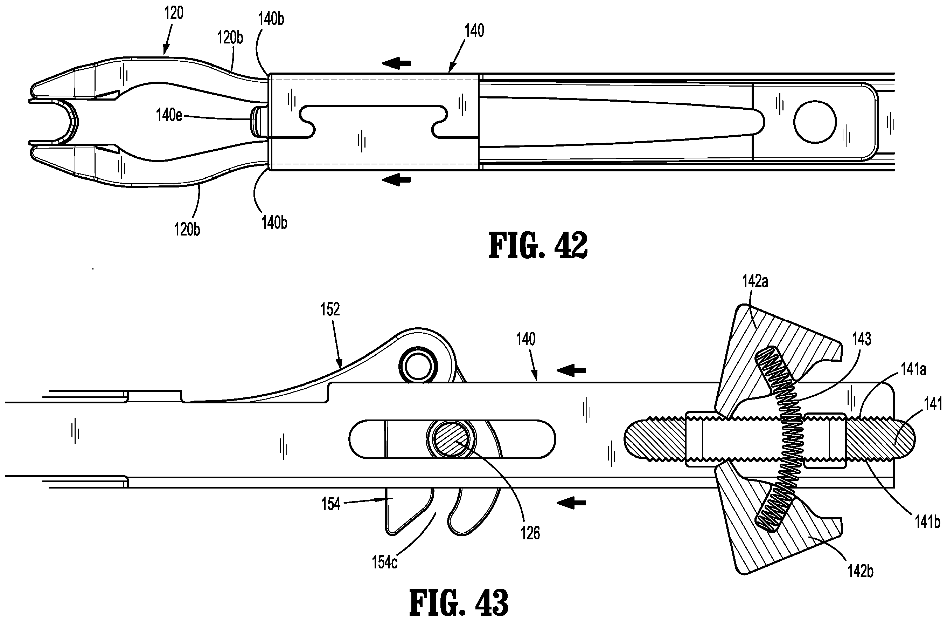

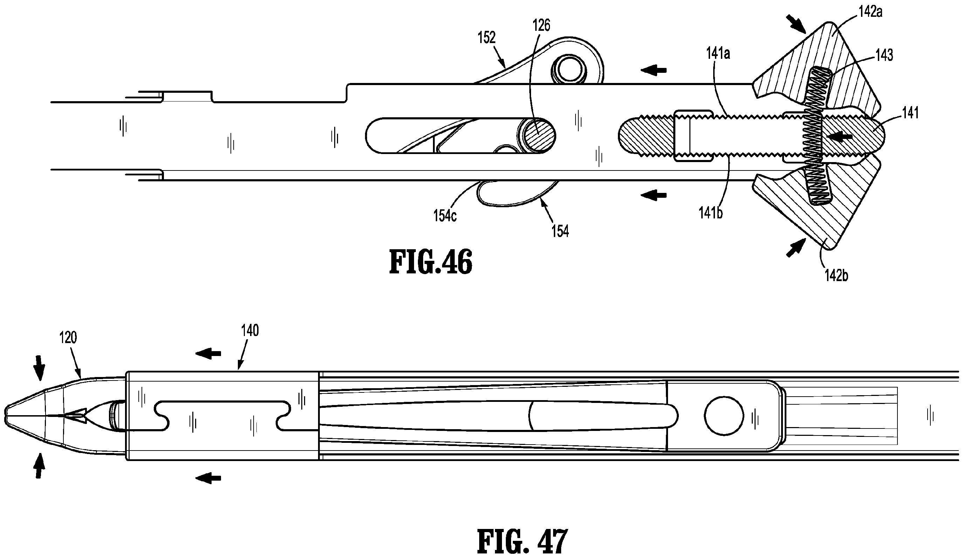

Ratchet mechanism 144 includes a snap-over ratchet spring 143, in the form of a coil spring, interposed between pawls 142a, 142b and extending through and across rack member 141. Snap-over spring 143 functions to maintain the teeth of pawls 142a, 142b in engagement with the respective rack teeth 141a, 141b of rack member 141 as rack member 141 is axially translated. When pawls 142a, 142b are in a first position (permitting drive channel 140 to move in a distal direction), snap-over spring 143 bulges or buckles in a proximal direction, as seen in FIGS. 36 and 43, and when pawls 142a, 142b are in a second position (permitting drive channel 140 to move in a proximal direction), snap-over spring 143 bulges or buckles in a distal direction, as seen in FIG. 46.

In operation, when drive channel 140 is moved to the distal-most position, a surface of rack member 141 engages distally bulging snap-over spring 143 to reverse the direction of buckling thereof, and thus reverse or change the orientation of pawls 142a, 142b. Additionally, when drive channel 140 is moved to the proximal-most position and rack teeth 141a, 141b of rack member 141 have once again cleared pawls 142a, 142b, another surface of rack member 141 engages proximally bulging snap-over spring 143 to again reverse the direction of buckling thereof, and thus again reverse or change the orientation of pawls 142a, 142b.

Clip applier 100 is provided with audible/tactile indication or feedback when pawls 142a, 142b are flipped at either end of the stroke of drive channel 140. In particular, when pawls 142a, 142b flip pawls 142a, 142b are accelerated by snap-over spring 143 and slap against the pair of walls 104c₁, 104c₂ of housing 104, thereby providing the user feedback that end of a stroke has been reached.

As seen in FIGS. 1-4 and FIGS. 10, 34 and 35, clip applier 100 includes a pair of jaws 120 mounted on or at a distal end of channel assembly 108 and actuatable by handles 106 of handle assembly 102. The pair of jaws 120 are formed of a suitable biocompatible material such as, for example, stainless steel or titanium.

The pair of jaws 120 are mounted in a distal end of outer channel 132 via a pin or a rivet 124 extending through the reciprocation limiting slot 140d of drive channel 140 such that jaws 120 are longitudinally stationary relative to outer channel 132 and drive channel 140. The pair of jaws 120 includes a first jaw member 121a, and a second jaw member 121b, wherein first jaw member 121a extends through first distal-facing openings 140e₁ of drive channel 140, and wherein second jaw member 121b extends through first distal-facing openings 140e₂ of drive channel 140. In this manner, tongue 140e of drive channel 140 extends between first jaw member 121a, and second jaw member 121b.

As seen in FIG. 4, the pair of jaws 120 defines a channel 120a therebetween for receipt of a surgical clip "C1" therein.

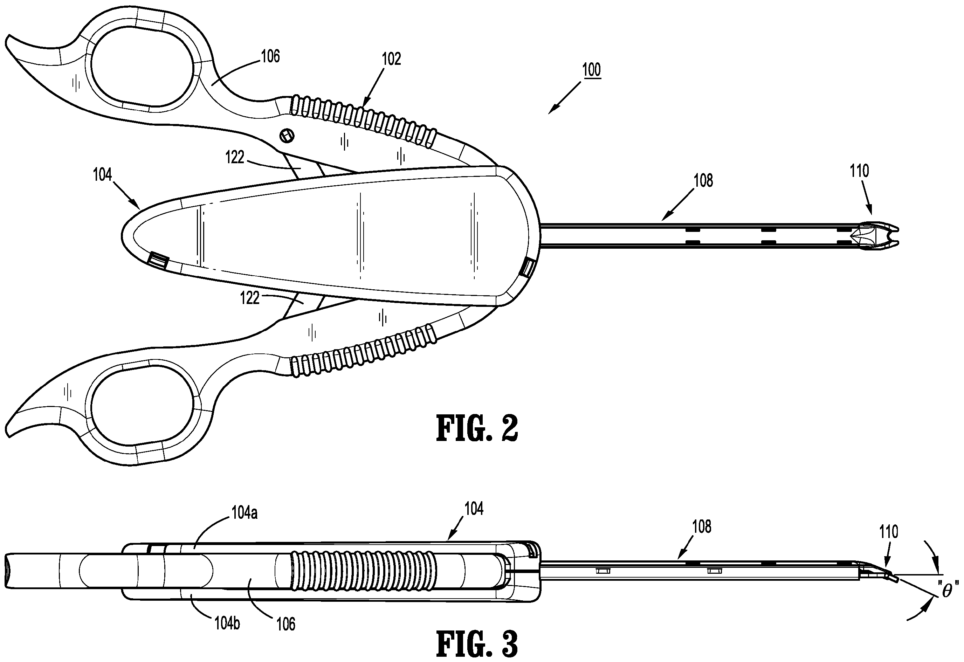

As seen in FIG. 3, a distal end of each jaw member 121a, 121b is angled at an angle "θ" of approximately between 15°-22.5° relative to a longitudinal axis of channel assembly 108 to enable an end user to better visualize a surgical clip disposed in the pair of jaws 120 by altering a line of sight of the end user to jaw members 121a, 121b of the pair of jaws 120. In this manner, the end user may better visualize and verify a presence of a surgical clip in the pair of jaws 120 while the surgical clip is being placed on or over a vessel.

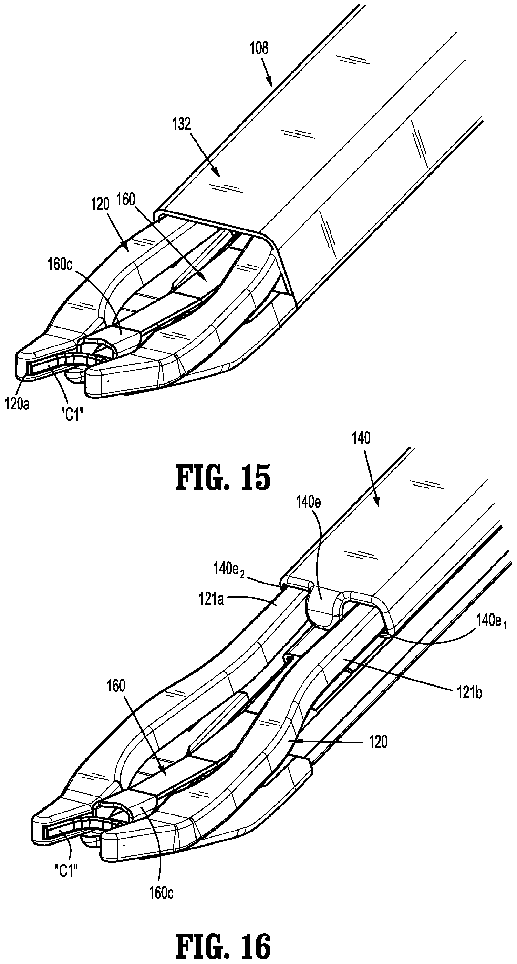

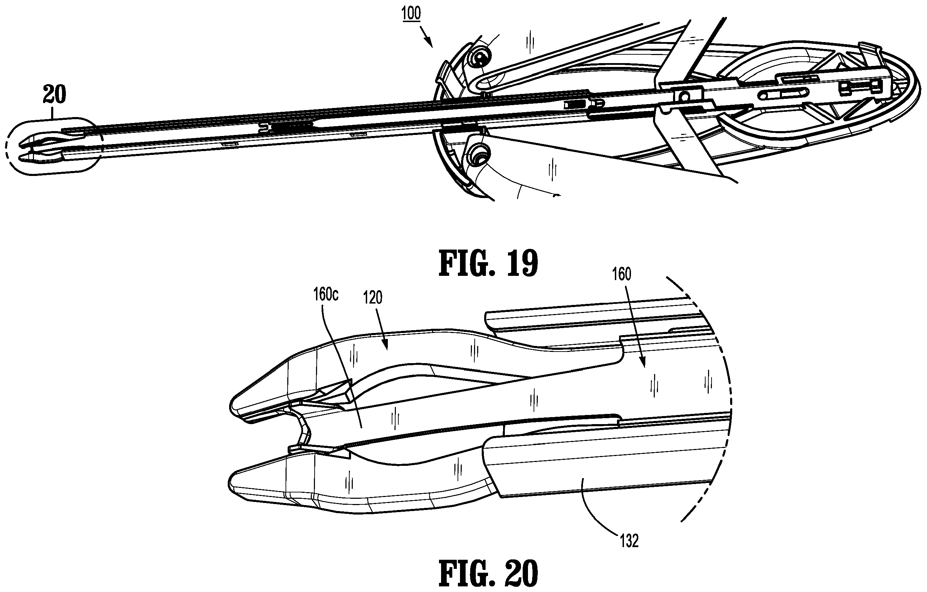

As seen in FIG. 4 and FIGS. 6, 15, 16, 19 and 20, clip applier 100 includes a clip pusher bar 160 slidably disposed against drive channel 140, at a location between drive channel 140 and cartridge cover 130. Pusher bar 160 includes a distal end 160a defining a pusher 160c configured and adapted to selectively engage/move a distal-most surgical clip "C1" stored in surgical clip applier 100. Pusher bar 160 further includes a proximal end 160b operatively connected to drive channel 140. Pusher bar 160 further defines an elongate window 160d therein for operative receipt of a proximal end of a constant force spring 176 therein, as will be discussed in greater detail below.

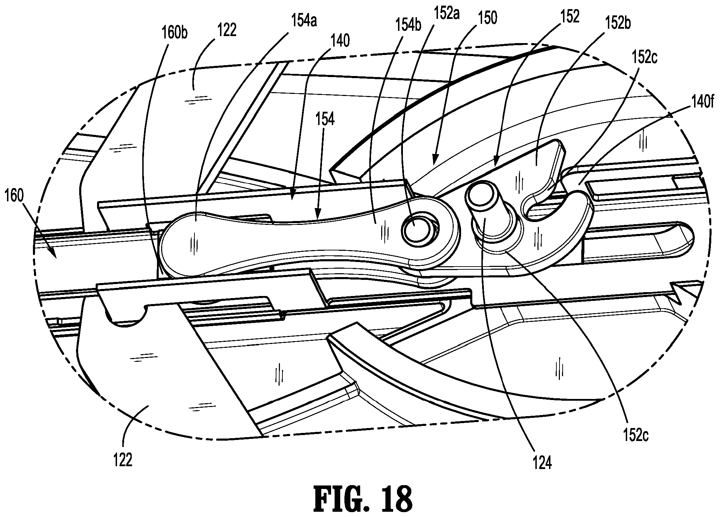

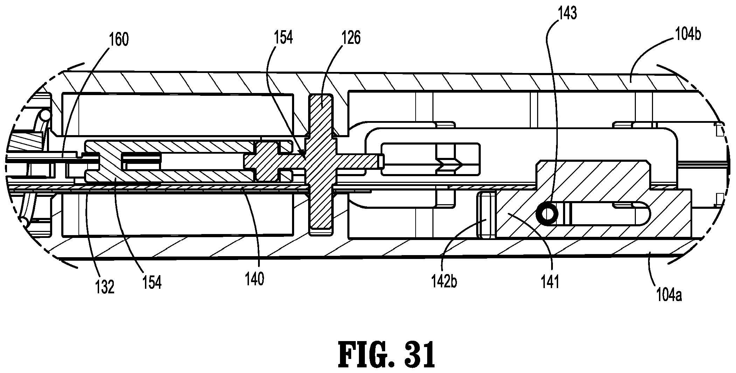

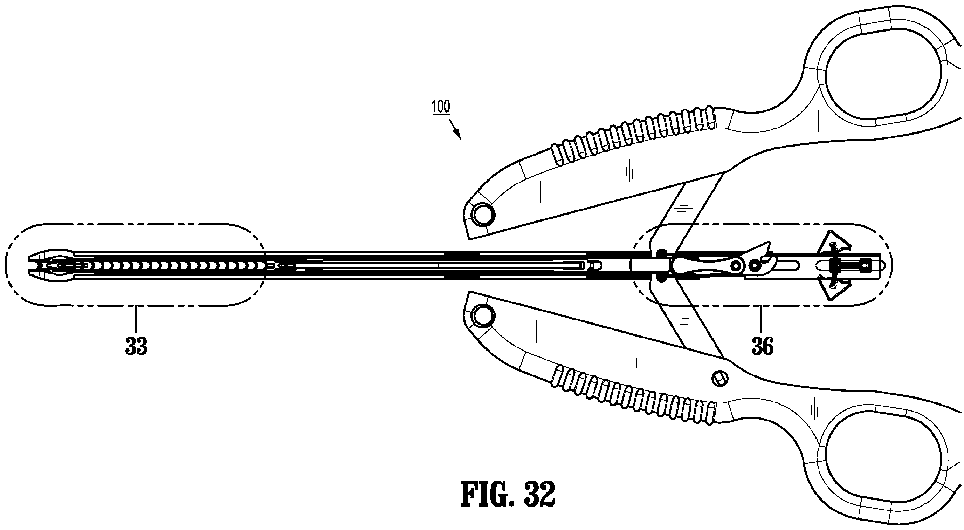

As seen in FIG. 4 and FIGS. 13, 18, 31 and 32, clip applier 100 further includes linkage mechanism 150 having a distal, driven link arm 154 having a first, distal end 154a pivotally connected to proximal end 160b of pusher bar 160. Linkage mechanism 150 further includes a proximal, driver link arm 152 having a first, distal end 152a pivotally connected to a second, proximal end 154b of driven link arm 154, and a second, proximal end 152b defining an arcuate slot 152c configured to slidably receive a tab 140f of drive channel 140. Arcuate slot 152c is open ended and is only engaged by tab 140f of drive channel 140 during select portions of a distal and proximal translation thereof, as will be described in greater detail below. Driver link arm 152 defines a central eyelet 152c for pivotal connection to a pin 126, such that driver link arm 152 is axially fixed with respect to housing 104.

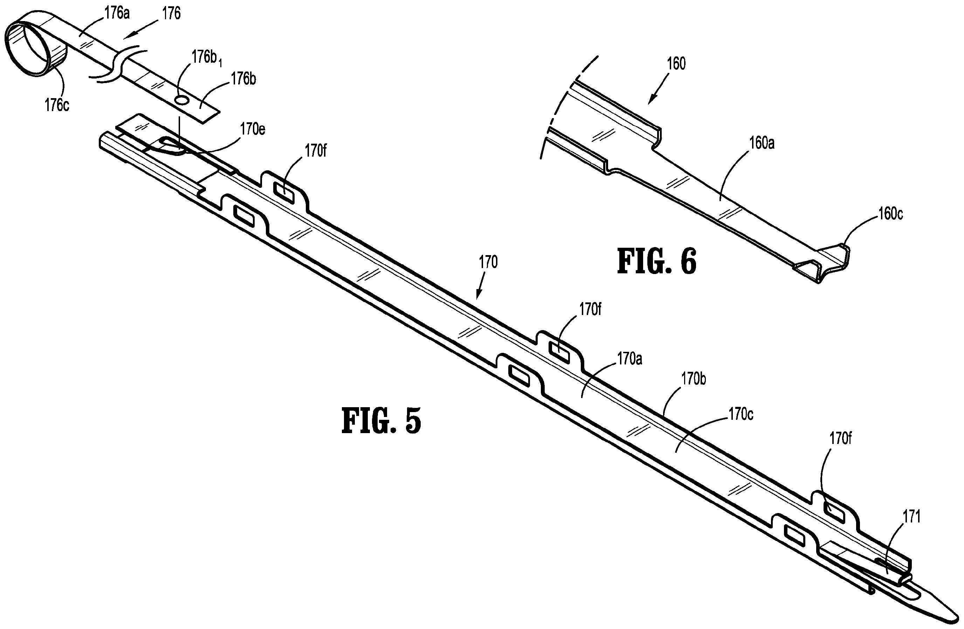

As seen in FIG. 4 and FIG. 5, clip applier 100 further includes a clip carrier 170 fixedly disposed within channel assembly 108 and disposed against pusher bar 160, at a location between pusher bar 160 and cartridge cover 130. Clip carrier 170 is generally a U-shaped channel structure having a lower wall 170a and a pair of side walls 170b defining a channel 170c between lower wall 170a and cartridge cover 130.

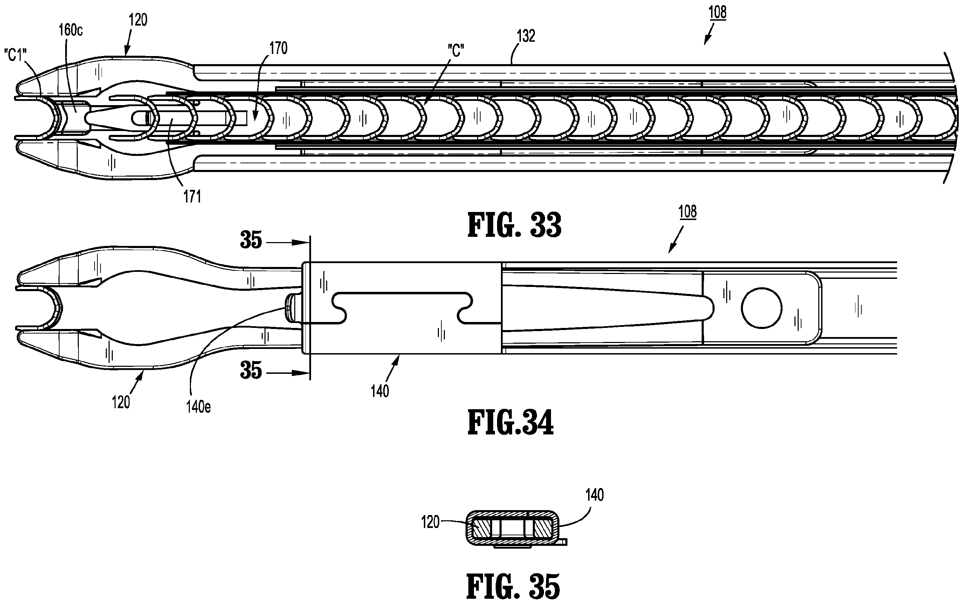

As seen in FIG. 4 and FIG. 33, a stack of surgical clips "C" is loaded and/or retained within channel 170c defined by clip carrier 170 and cartridge cover 130 in a manner so as to slide therewithin and/or therealong. Clip carrier 170 is configured and dimensioned to slidably retain the stack or plurality of surgical clips "C" in tip-to-tail fashion therewithin.

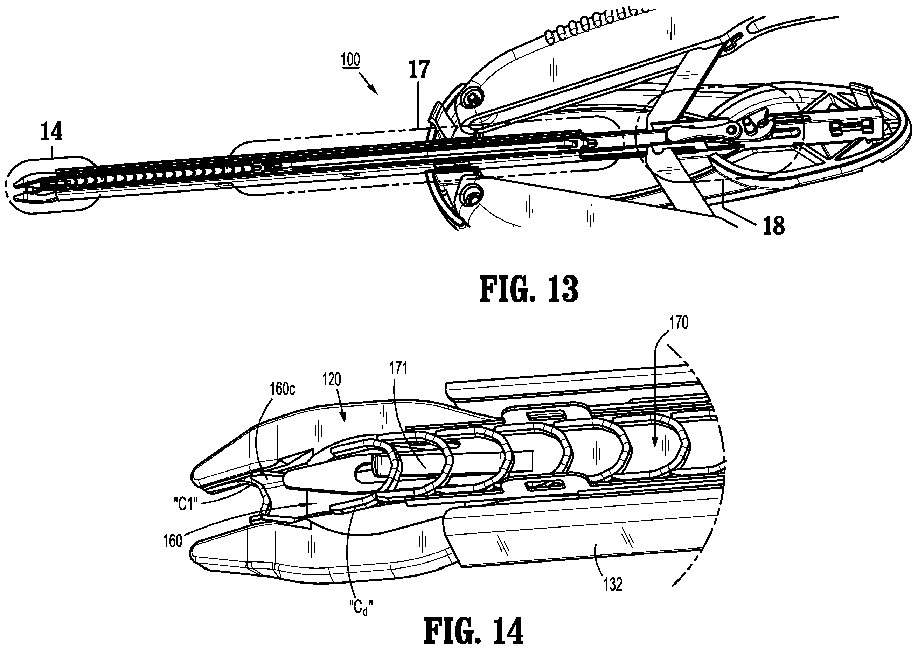

As seen in FIG. 5 and FIG. 14, a distal end of clip carrier 170 includes a resilient central tang 171. Tang 171 is configured and adapted to selectively engage a backspan of a distal-most surgical clip "C_{d}" of the stack of surgical clips "C" retained within carrier 170 to thereby prevent the stack of surgical clips "C" within channel 170c of clip carrier 170.

As seen in FIG. 4 and FIGS. 7-9 and 29, clip applier 100 further includes a clip follower 174 at least partially slidably disposed within channel 170c of clip carrier 170. As will be discussed in greater detail below, clip follower 174 is positioned behind the stack of surgical clips "C" and is provided to urge the stack of surgical clips "C" forward during an actuation of clip applier 100. As will also be described in greater detail below, clip follower 174 is actuated by a constant force spring 176 upon the advancement, by the pusher bar 160, of the distal-most surgical clip "C_{d}" distally passed the tang 171 of the clip carrier 170, during a firing of clip applier 100.

Clip follower 174 includes an elongate body 174a having a distal end portion 174b configured and dimensioned for passage through channel 170c of clip carrier 170. Distal end portion 174b of clip follower 174 is configured to seat against a backspan of a proximal-most clip "Cₚ" of the stack of surgical clips "C".

Clip follower 174 includes a proximal end portion 174c folded over onto itself to define a tail 174d such that proximal end portion 174c is in the form of a leaf spring. Proximal end portion 174c defines an upper window 174a₁ formed in a proximal portion of elongate body 174a, and a lower window 174d₁ formed in tail 174d and overlying or in registration with upper window 174a₁. Clip follower 174 includes a tab 174e extending between elongate body 174a and tail 174d.

Clip follower 174 is fabricated from a resilient material such that tail 174d of clip follower 174 is lightly spring-biased against a surface of drive channel 140.

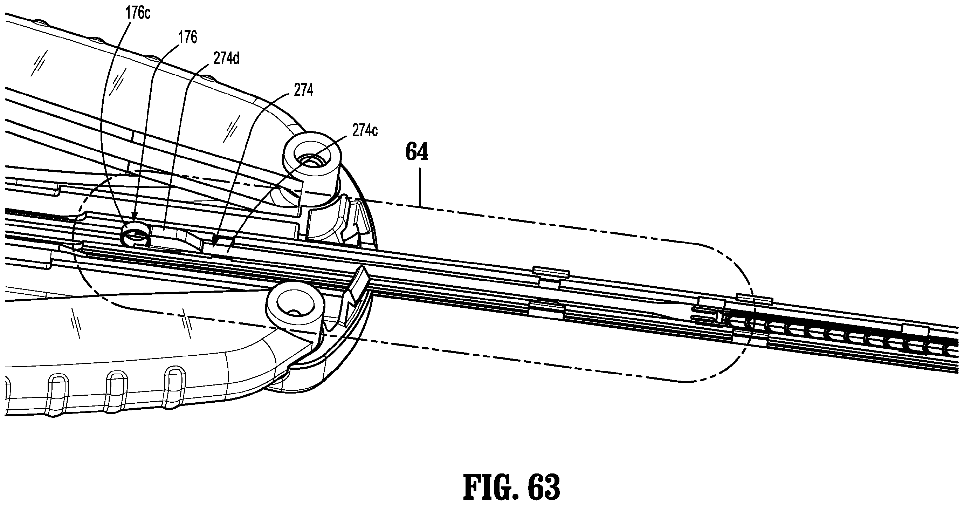

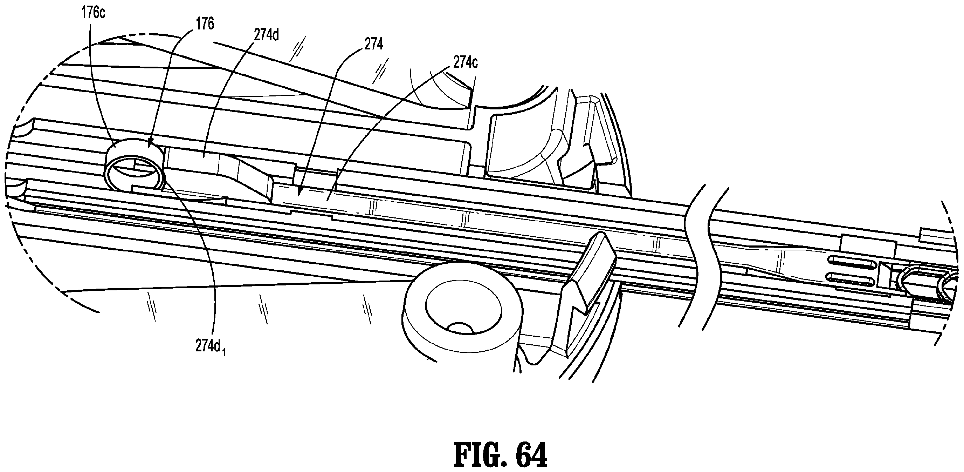

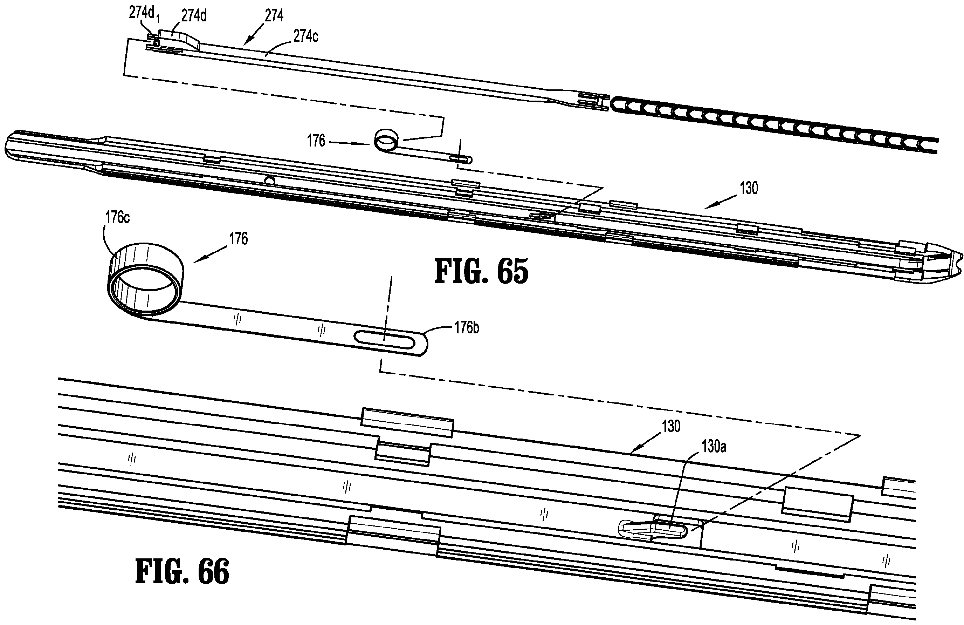

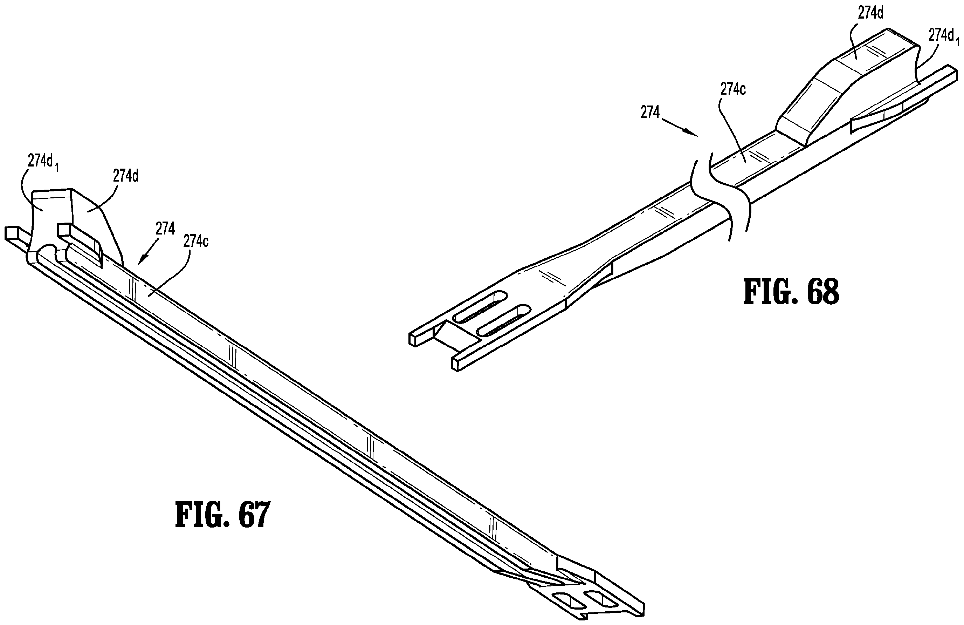

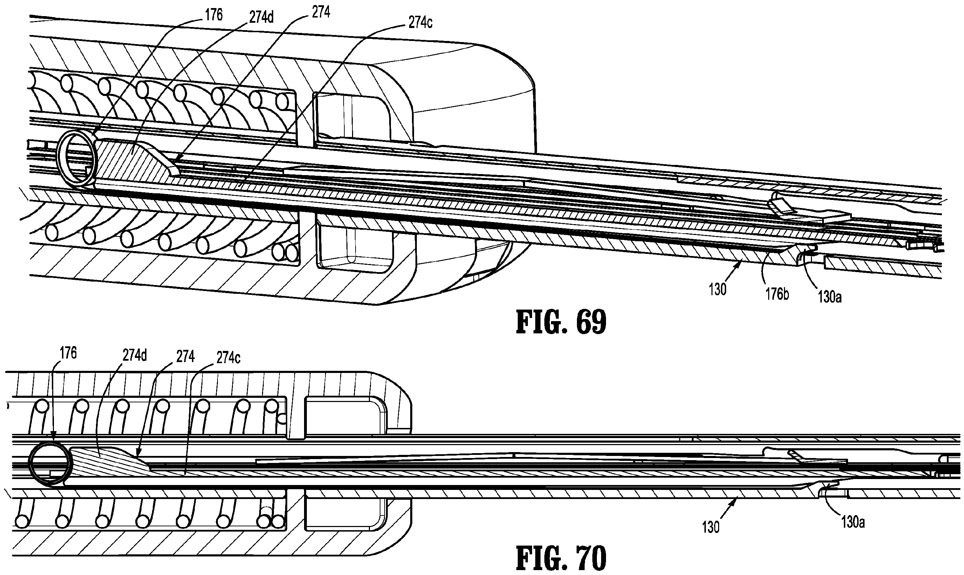

Turning momentarily to FIGS. 63-70, an alternate embodiment of a clip follower 274 is shown and will be described. Clip follower 274 is substantially similar to clip follower 174 and thus will only be described in detail herein to the extent necessary to describe differences in construction and functions thereof. As seen in FIGS. 63-65 and 67-70, clip follower 274 includes a proximal fin 274d projecting transversely from a proximal end portion 274c. Proximal fin 274d defines a proximal surface 274d₁ having a concave arcuate profile configured to receive and seat with a coiled or spooled portion 176c of a constant force spring 176.

Turning now to and as seen in FIGS. 4, 5 and 9, clip applier 100 includes a constant force spring 176 supported in handle assembly 104 and channel assembly 108. Constant force spring 176 is in the form of a ribbon including a body portion 176a having a distal end 176b, and a proximal end 176c coiled onto itself to form a spool.

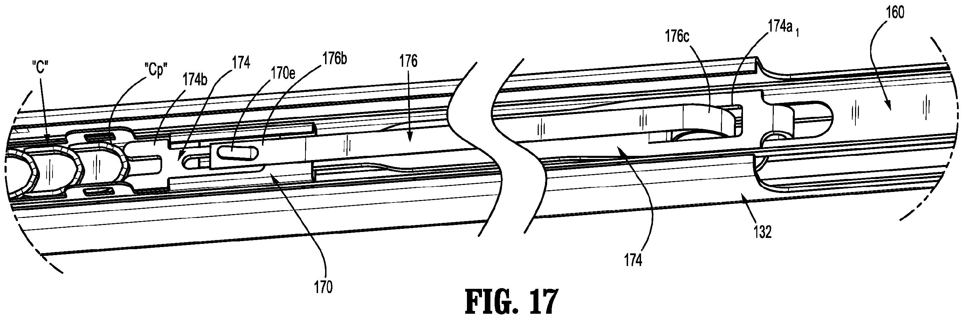

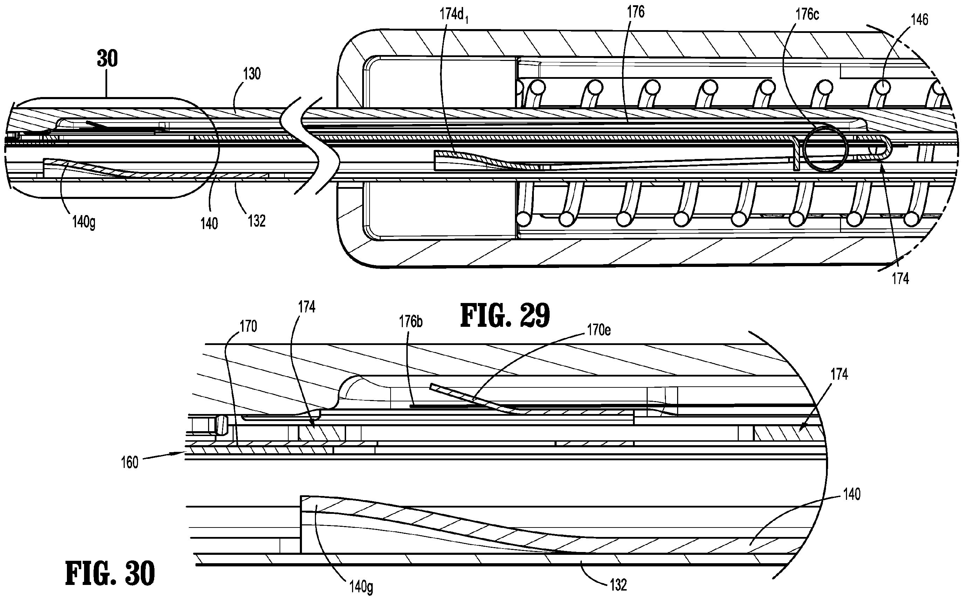

Body portion 176a and distal end 176b of constant force spring 176 are disposed within a channel formed in an underside of cartridge cover 130, wherein body portion 176a and distal end 176b of constant force spring 176 are interposed between cartridge cover 130 and elongate body 174a of clip follower 174. Distal end 176b of constant force spring 176 is secured to tine 170e of clip carrier 170, as described below. In particular, as seen in FIGS. 17 and 30, distal end 176b of constant force spring 176 defines an opening 176b₁ (See FIG. 5) that is slipped over a distally extending tine 170e of clip channel 170. In this manner, tine 170e of clip channel 170 prevents distal end 176b of constant force spring 176 from moving proximally.

Turning now momentarily to FIGS. 63-66 and 69-70, in an alternate embodiment, it is contemplated that distal end 176b of constant force spring 176 may be hooked onto a hook or tine 130a projecting distally and inwardly from an inner surface of cartridge cover 130.

Proximal coiled or spooled end 176c of constant force spring 176 is disposed within lower window 174a₁ and upper window 174d₁ of follower 174, wherein proximal coiled or spooled end 176c is interposed between tab 174e of follower 174 and a proximal end-most wall of follower 174. Due to a memory of constant force spring 176, proximal coiled or spooled end 176c thereof tends to want to roll-up along body portion 176a.

Constant force spring 176 is a pre-stressed flat strip of spring material which is formed into a virtually constant radius coil, wherein distal end 176b of constant force spring 176 is secured to clip channel 170 as described above, and wherein proximal coiled or spooled end 176c of constant force spring 176 is disposed within proximal end portion 174c of clip follower 174 as described above.

Constant force spring 176 functions to maintain a constant pressure or distal force on the stack of surgical clips "C" such that the stack of surgical clips "C" are pressed against resilient central tang 171 of clip carrier 170. In this manner, in operation, as will be discussed in greater detail below, the stack of surgical clips "C" advances distally on demand as the distal-most surgical clip "C_{d}" is advanced past resilient central tang 171 by pusher bar 160.

As seen in FIGS. 24, 57 and 58, clip applier 100 includes a lockout for preventing actuation of the pair of jaws 120 following an application of the last surgical clip of the stack of surgical clips "C". The lockout includes a stop tab or bump 174d₂ formed in distally oriented tail 174d of clip follower 174, and a stop tab or bump 140g projecting proximate a proximal end of drive channel 140. An operation of the lockout will be described in greater detail below.

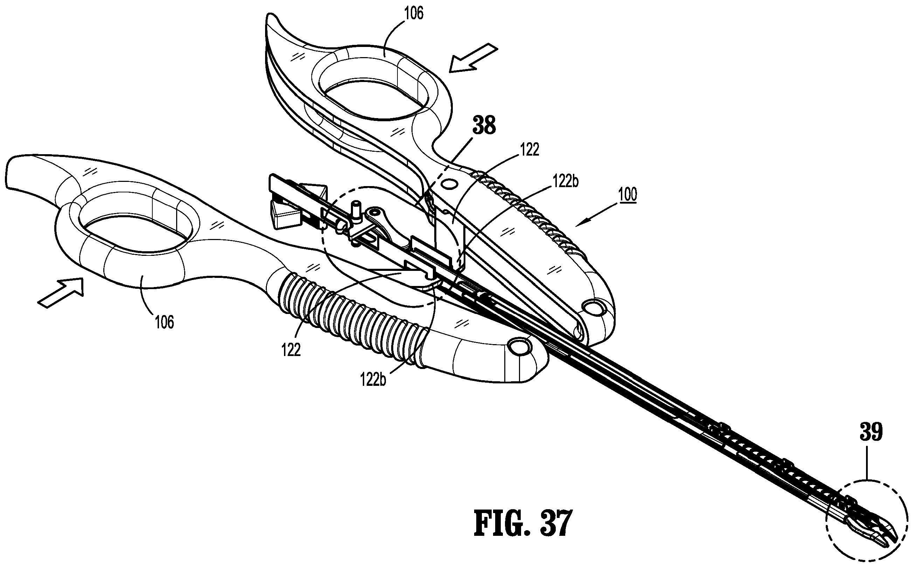

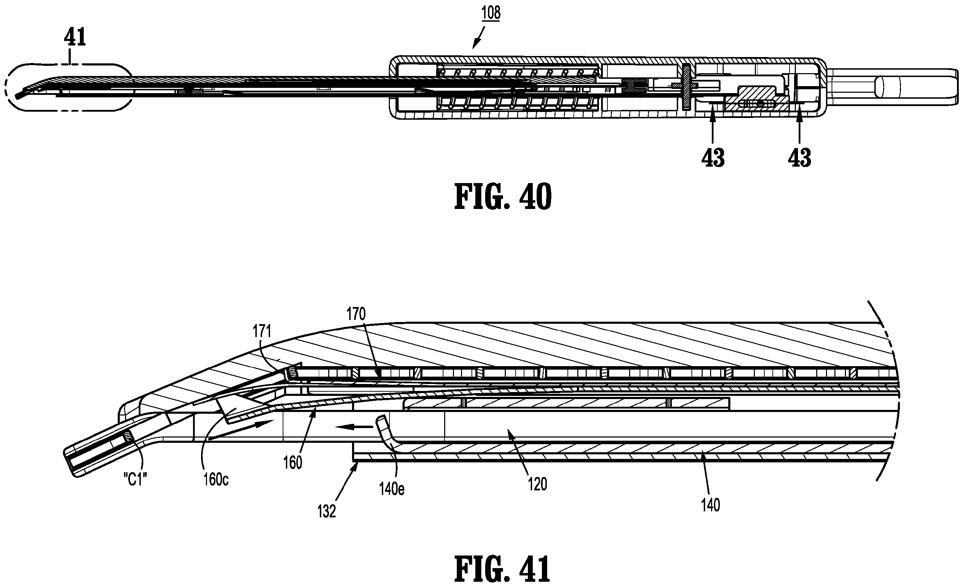

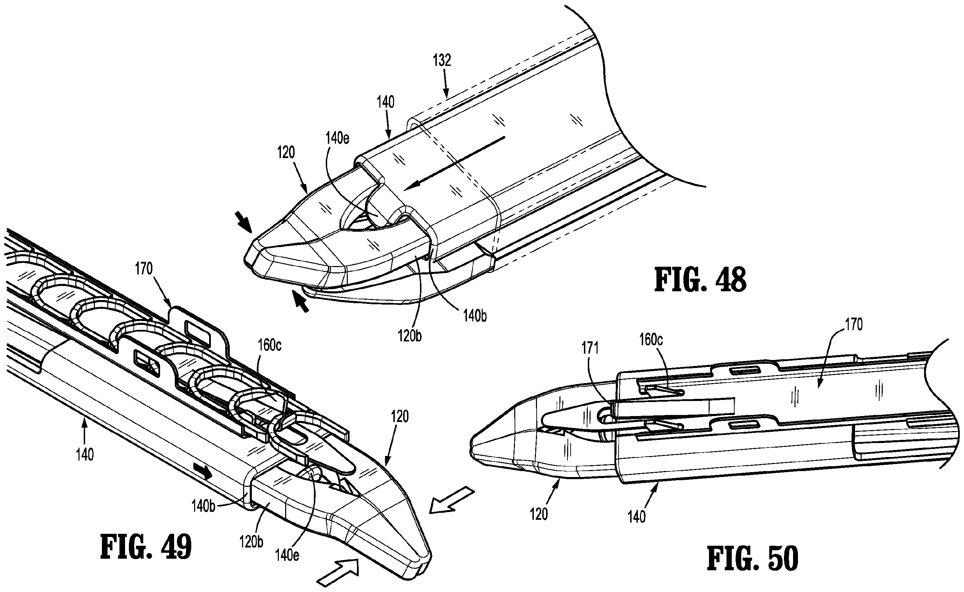

With reference to FIGS. 1-58, and particularly to FIGS. 37-58, the operation of the Covidien Clip Applier 100 is provided. Prior to any initial squeezing of handles 106 of clip applier 100 (with clip applier 100 including a full stack of surgical clips "C"), as seen in FIGS. 12-36, drive channel 140 is located at a proximal-most position, pusher bar 160 is located at a distal-most position, and clip follower 174 is located at a proximal-most position. When pusher bar 160 is located at the distal-most position, pusher 160c thereof supports any surgical clip disposed between the pair of jaws 120.

With drive channel 140 located at a proximal-most position and pusher bar 160 located at a distal-most position, linkage mechanism 150 is situated in an un-actuated position, wherein driven link arm 154 is substantially aligned with a longitudinal axis of channel assembly 108.

Also prior to the initial squeeze, if no surgical clips "C" are present within the pair of jaws 120, a surgical clip "C" is first loaded into the pair of jaws 120 during an initial squeezing of handles 106 or a priming of clip applier 100 (to load an initial surgical clip into the pair of jaws 120).

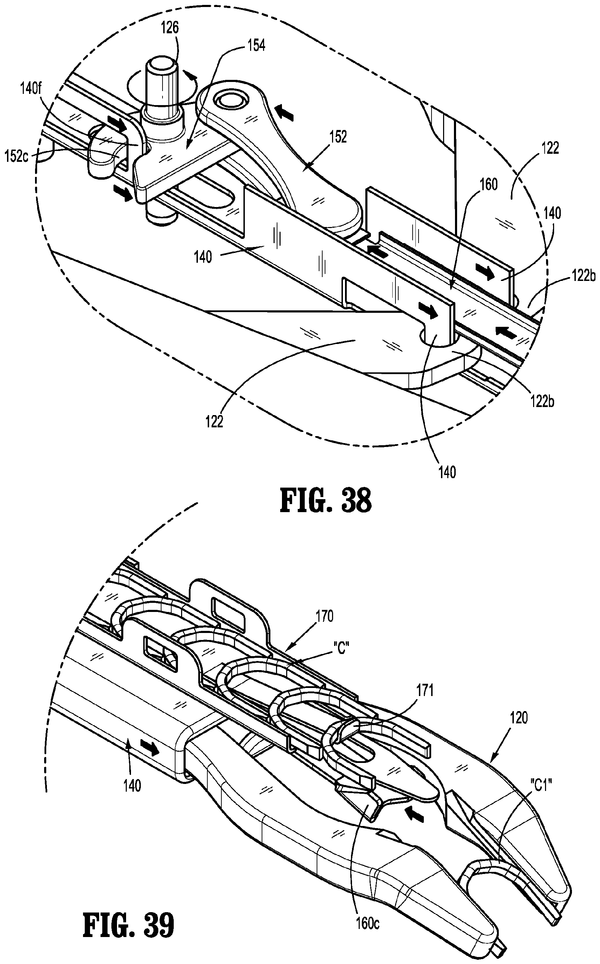

As seen in FIGS. 37-43 and particularly FIGS. 37 and 38, during a first stage of an initial squeezing of handles 106 or priming of clip applier 100, second ends 122b of link members 122 are caused to be moved distally relative to housing 104. As second ends 122b of link members 122 are moved distally, link members 122 act on drive channel 140 to transmit distal axial movement to drive channel 140.

As seen in FIGS. 38 and 43 and FIG. 42, as drive channel 140 is moved distally, during a second stage of an initial squeeze of handles 106, following an initial dwell period, upon a further squeezing of handles 106, tab 140f of drive channel 140 enters into arcuate slot 152c of driver link arm 152 and acts on driver link arm 152 to rotate driver link arm 152 about pin 126.

As driver link arm 152 is rotated about pin 126, distal end 152a of driver link arm 152 acts on proximal end 154b of driven link arm 154 to pull driven link arm 154 in a proximal direction.

As seen in FIGS. 39-41, as driven link arm 154 is pulled in a proximal direction, distal end 154a of driven link arm 154 acts on pusher bar 160 to retract pusher bar 160 in a proximal direction. The relative dimensions and configurations of arcuate slot 152c of driver link arm 152, the radii of rotation of distal end 152a and proximal end 152b of driver link arm 152, and the relative length of driven link arm 154 determines when pusher bar 160 is retracted a sufficient amount such that pusher 160c is disposed proximal of a distal-most surgical clip "C1".

Following the retraction of pusher bar 160 by an amount sufficient that pusher 160c is disposed proximal of a distal-most surgical clip "C1", tab 140f of drive channel 140 exits arcuate slot 152c of driver link arm 152 such that proximal movement of pusher bar 160 is halted and distal movement of drive channel 140 continues.

During the initial squeeze of handles 106, pawls 142a, 142b function to create an audible click and/or a tactile vibration, thereby indicating to the user that handles 106 of surgical clip applier 100 have gone through at least a portion of a stroke. Additionally, cartridge cover 130 is fabricated from a transparent material, allowing the user to clearly see the clips "C" in the stack of clips.

As described above, the first audible/tactile indication, by pawls 142a, 142b, indicates to the user that a surgical clip "C" has been appropriately loaded.

As seen in FIG. 43, also during the first stage of the initial squeeze of handles 106, as drive channel 140 is moved in a distal direction, rack member 141 of ratchet mechanism 144 is moved distally causing teeth 141a thereof to move into engagement with and over or across a tooth of each pawl 142a, 142b. Once rack member 141 of ratchet mechanism 144 is moved into engagement with each pawl 142a, 142b, drive channel 140 can not return to a home or proximal-most position until rack member 141 has cleared pawls 142a, 142b.

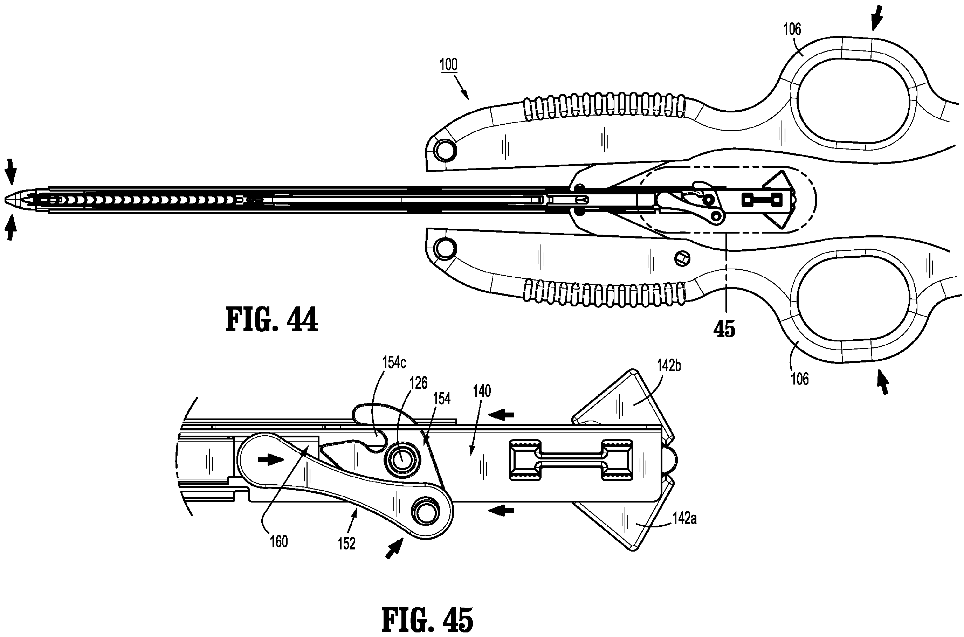

During the second stage of the initial squeeze of handles 106, as seen in FIGS. 44-51, drive channel 140 is moved distally until a distal edge of side walls 140b of drive channel 140 engages against outer camming surfaces 120b of the pair of jaws 120 thus causing the pair of jaws 120 to approximate toward one another (and to form surgical clip "C1" interposed therebetween if a surgical clip is present).

Additionally, during the second stage of the initial squeeze of handles 106, as handles 106 are squeezed to distally advance drive channel 140, drive channel 140 and/or link members 122 act(s) on biasing member 146 to compress biasing member 146 against housing 104.

Referring to FIGS. 44-51, clip applier 100 is illustrated following a completed initial stroke or squeezing of handles 106 and during an opening of handles 106. In this condition, drive channel 140 is at a distal-most position, pusher bar 160 is at a proximal-most position, biasing member 146 is in a compressed condition, and each pawl 142a, 142b is located proximal of rack member 141.

In use, handles 106 may be opened by hand, or the closure force tending to maintain handles 106 closed is removed, whereby the compressed biasing member 146 may expand. As compressed biasing member 146 expands, biasing member 146 acts on link arms 122 and/or drive channel 140 to assist in the opening of handles 106 and the return of clip applier 100 to an open or initial condition.

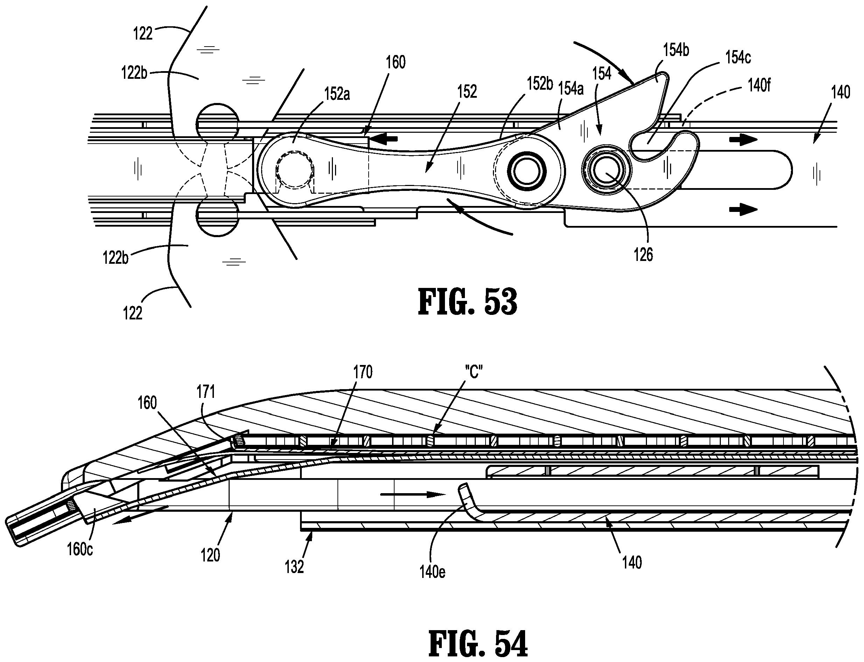

As seen in FIGS. 53-54, during an opening of handles 106, second ends 122b of link members 122 are caused to be moved proximally relative to housing 104. As second ends 122b of link members 122 are moved proximally, link members 122 act on drive channel 140 to transmit proximal axial movement to drive channel 140.

As drive channel 140 is moved proximally, following an initial dwell period, tab 140f of drive channel 140 re-enters into arcuate slot 152c of driver link arm 152 and acts on driver link arm 152 to rotate driver link arm 152 about pin 126 in a reverse direction.

As driver link arm 152 is rotated about pin 126 (in a reverse direction), distal end 152a of driver link arm 152 acts on proximal end 154b of driven link arm 154 to push driven link arm 154 in a distal direction.

As driven link arm 154 is pushed in a distal direction, distal end 154a of driven link arm 154 acts on pusher bar 160 to advance pusher bar 160 in a distal direction, as seen in FIGS. 53 and 54. The relative dimensions and configurations of arcuate slot 152c of driver link arm 152, the radii of rotation of distal end 152a and proximal end 152b of driver link arm 152, and the relative length of driven link arm 154 determines when pusher bar 160 is advanced a sufficient amount such that pusher 160c of pusher bar 160 loads a distal-most surgical clip "C1" of the stack of surgical clips "C" into the pair of jaws 120.

In particular, as pusher bar 160 is advanced in a distal direction, pusher 160c thereof engages a backspan of distal-most clip "C1" and begins to move or urge distal-most clip "C1" distally out of clip carrier 170 and into the pair of jaws 120. As distal-most clip "C1" is moved distally, central tang 171 of clip carrier 170 is momentarily deflected or cammed out of engagement with distal-most clip "C1" and returned to its un-deflected or un-cammed state to capture a subsequent surgical clip of the stack of surgical clips "C". During the opening of handles 106, pusher bar 160 is advanced an amount sufficient to place distal-most surgical clip "C1" in channels 120a of pair of jaws 120.

As pusher 160c of pusher bar 160 advanced distal-most surgical clip "C1" into the pair of jaws 120, the remaining stack of surgical clips "C" is advanced distally due to an advancement force acting on the stack of surgical clips "C" by clip follower 174. In particular, with the removal of the distal-most surgical clip "C1", proximal coiled end 176c of constant force spring 176 continues to coil up onto itself, thus shortening a length of body portion 176a since distal end 176b of constant force spring 176 is anchored to clip carrier, as described above.

As proximal coiled end 176c of constant force spring 176 coils up onto itself, proximal coiled end 176c acts on tabs 174e of clip follower 174 to exert a distal force on clip follower 174 and distally advance clip follower 174. Clip follower 174 is advanced distally until the stack of surgical clips "C" is stopped by central tang 171 of clip carrier 170.

Following the return of pusher bar 160 to the distal-most position, tab 140f of drive channel 140 exits arcuate slot 152c of driver link arm 152 such that distal movement of pusher bar 160 is halted and proximal movement of drive channel 140 continues.

With pusher bar 160 at the distal-most position, pusher 160c of pusher bar 160 remains against the backspan of the distal-most surgical clip "C1" that was loaded into the pair of jaws 120. In this manner, when the pair of jaws 120 are advanced over a target vessel, pusher 160c of pusher bar 160 supports the backspan of the distal-most surgical clip "C1" to inhibit the distal-most surgical clip "C1" from backing out of the pair of jaws 120.

During the complete opening of handles 106, the proximal-most positioning of drive channel 140 causes pawls 142a, 142b to flip and snap, creating an audible click and/or a tactile vibration, thereby indicating to the user that handles 106 of surgical clip applier 100 have completely opened and that surgical clip applier 100 has undergone a complete cycle.

As seen in FIG. 46, also during the opening of handles 106, as drive channel 140 is moved in a proximal direction, rack member 141 of ratchet mechanism 144 is moved proximally causing teeth 141a thereof to move back into engagement with and over or across a tooth of each pawl 142a, 142b. Once rack member 141 of ratchet mechanism 144 is moved back into engagement with each pawl 142a, 142b, drive channel 140 can not be re-advanced distally to an advanced or distal-most position until rack member 141 has re-cleared pawls 142a, 142b.

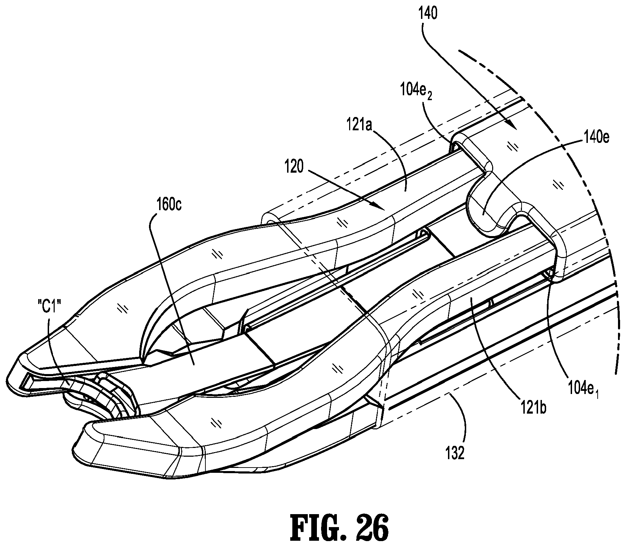

During the opening of handles 106, as seen in FIG. 26, drive channel 140 is moved proximally such that and until tongue 140e of drive channel 140 engages against inner camming surfaces 120c of the pair of jaws 120 thus assisting and/or causing the pair of jaws 120 to spread apart from one another to a fully open condition (in the event that the pair of jaws 120 were being held in an approximated condition by some external pressure or force).

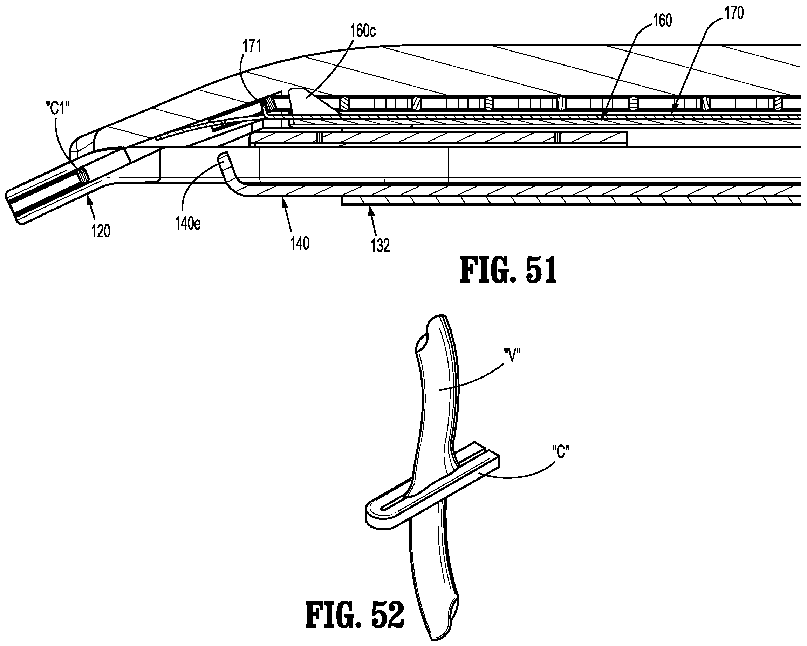

Following an initial priming of clip applier 100, any further squeezing of handles 106 will result in the firing of clip applier 100 to apply a surgical clip "C1" onto a vessel "V" or any other biological tissue, as seen in FIG. 52.

During a re-squeezing of handles 106 as drive channel 140 is moved distally and pusher bar 160 is moved proximally, as described above, with surgical clip "C1" loaded into the pair of jaws 120, as distal edge of side walls 140b of drive channel 140 engages against outer camming surfaces 120b of the pair of jaws 120, the pair of jaws 120 are approximated toward one another to form surgical clip "C1".

As seen in FIG. 52, surgical clip "C1" may be formed or crimped onto a vessel "V" or any other biological tissue. In certain instances, the surgeon may decide or need to fire a surgical clip "C1" onto a suture in lieu of typing a knot in the suture.

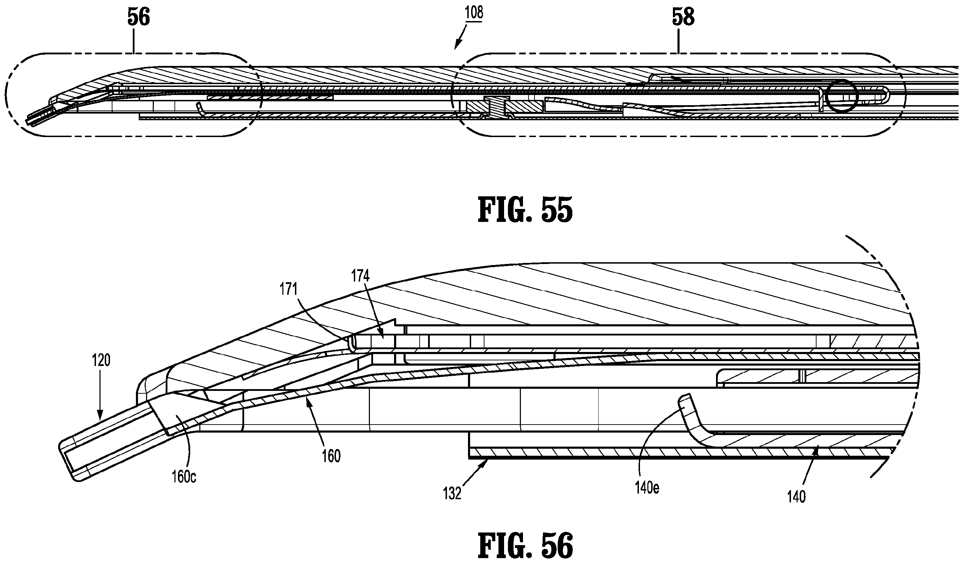

Turning now to FIGS. 55-56 and FIGS. 57-58, a distal end of clip applier 100 is illustrated following a complete stroke or squeezing of handles 106 and after a final surgical clip has been urged from clip carrier 170 and loaded into the pair of jaws 120, as described above. Following loading of the final surgical clip into the pair of jaws 120, clip follower 174 is advanced to a distal-most position by constant force spring 176, as described above.

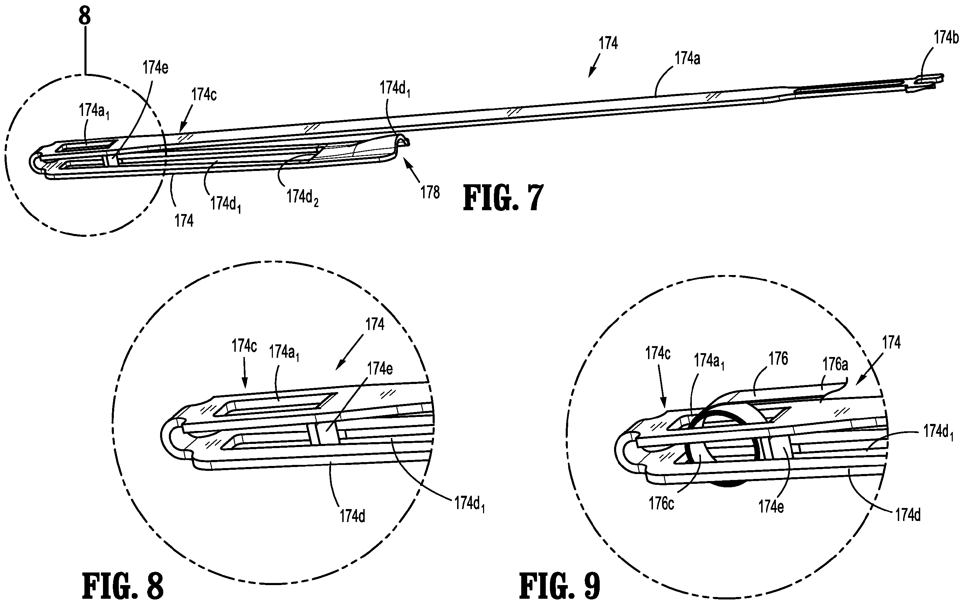

With the last clip loaded into the pair of jaws 120, as clip applier 100 is fired once again to fire the last clip, by squeezing handles 106, pusher bar 160 is moved in the proximal direction (as described above) such that pusher 160c (see FIG. 6) cams over and proximal of distal end portion 174b (see FIG. 7) of clip follower 174. Then, as handles 106 are opened, pusher bar 160 is moved in the distal direction (as described above). As pusher bar 160 is moved in the distal direction, pusher 160c acts on distal end portion 174b of clip follower 174 to urge clip follower 174 distally until (1) distal end portion 174b of clip follower 174 is disposed between the pair of jaws 120, and (2) front edge 174d₃ of lower window 174d₁ (see FIG. 7) of tail 174d of clip follower 174 is disposed distally of stop tab 140g of drive channel 140.

At this point, there is no clip in the pair of jaws 120, and the lockout tail 174d on clip follower 174 is engaged with stop tab 140g on drive channel 140. As such, if the user attempts to fire clip applier 100 at this point, drive channel 140 drives lockout tail 174d of clip follower 174 against the pair of jaws 120, and together with distal end portion 174b of clip follower 174 being disposed between the pair of jaws 120, clip applier 100 is incapable of having the pair of jaws 120 closed.

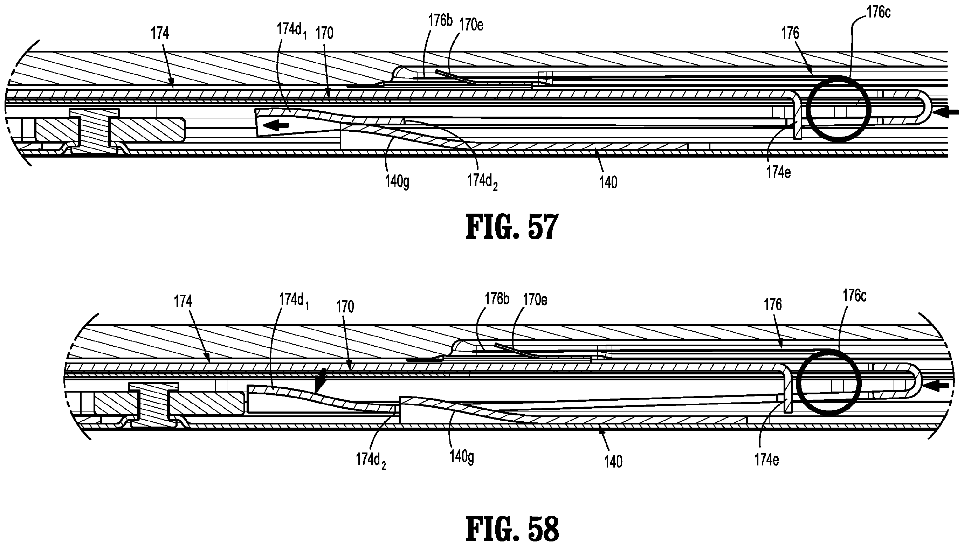

Additionally, with stop tab 140g of drive channel 140 disposed proximally of front edge 174d₃ of lower window 174d₁ of tail 174d of clip follower 174, as seen in FIG. 58, any attempt to re-actuate clip applier 100, whereby drive channel 140 is distally advanced, will result in stop tab 140g of drive channel 140 abutting against front edge 174d₃ of lower window 174d₁ of tail 174d of clip follower 174 thereby preventing distal advancement of drive channel 140.

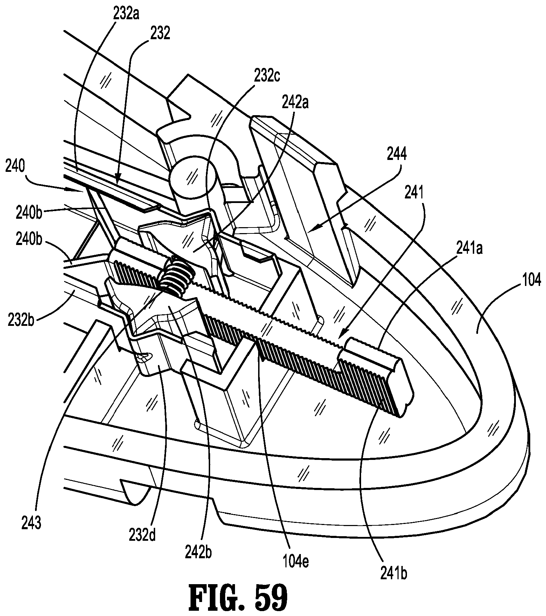

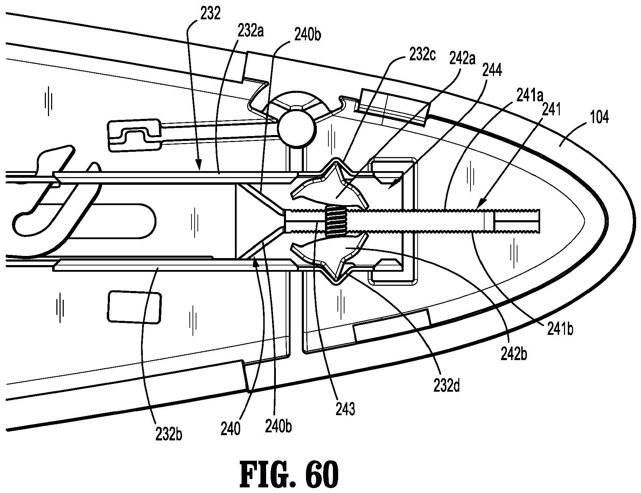

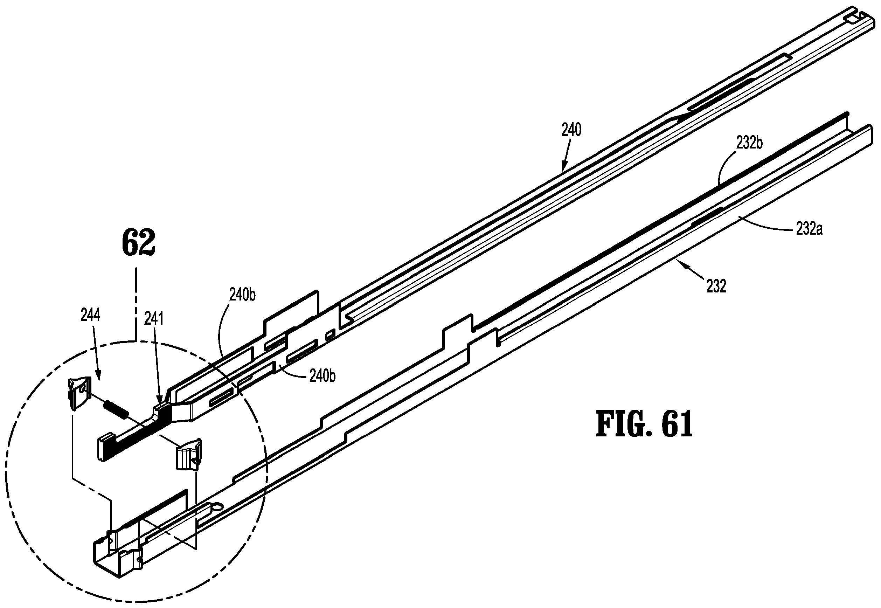

Turning now to FIGS. 59-62, a lower channel according to another embodiment of the present disclosure, is generally designated as 232. Lower channel 232 is substantially similar to lower channel 132 and thus will only be discussed in detail herein to the extent necessary to describe differences in structure and operation thereof. As seen in FIGS. 59-62, lower channel 232 includes a pair of opposed, upstanding, proximal side walls 232a, 232b. Each side wall 232a, 232b defines a substantially V-shaped channel 232c, 232d, wherein each V-shaped channel 232c, 232d extends in a direction orthogonal to a longitudinal axis of lower channel 232. A respective rib 232e, 232f extends into each V-shaped channel 232c, 232d.

With continued reference to FIGS. 59-62, a drive channel according to another embodiment of the present disclosure, is generally designated as 240. Drive channel 240 is substantially similar to drive channel 140 and thus will only be discussed in detail herein to the extent necessary to describe differences in structure and operation thereof. As seen in FIGS. 59-62, opposed side walls 240b thereof join together at a proximal end of drive channel 240 to form a tail or ratchet rack member 241. Ratchet rack member 241 includes a first set of teeth 241a, on a first side thereof, configured and adapted to engage with a first ratchet pawl 242a, and a second set of teeth 241b, on a second side thereof, configured and adapted to engage a second ratchet pawl 242b.

Ratchet rack member 241 of drive channel 240 extends between side walls 232a, 232b of lower channel 230 and through a slot 104e formed in housing 104. Additionally, first ratchet pawl 242a is interposed between side wall 232a of lower channel 230 and ratchet rack member 241 of drive channel 240, and second ratchet pawl 242b is interposed between side wall 232b of lower channel 230 and ratchet rack member 241 of drive channel 240.

Rack member 241 and pawls 242a, 242b define a ratchet mechanism 244. In use, as drive channel 240 is moved axially, rack member 241 is caused to be moved relative to first and second ratchet pawl 242a, 242b. In so doing, the series of rack teeth 241a, 241b have a length which allows respective pawls 242a, 242b to reverse and advance back over rack member 241 when rack member 241 changes between proximal and distal movement as drive channel 240 reaches a proximal-most or distal-most position.

Each pawl 242a, 242b is pivotally supported in lower channel 230 at a location wherein each pawl 242a, 242b is in substantial operative engagement with respective rack teeth 241a, 241b of rack member 241. Pawls 242a, 242b are engageable with rack member 241 to restrict longitudinal movement of rack member 241 and, in turn, drive channel 240.

Each pawl 242a, 242b has a substantially triangular profile, wherein a remote corner 242a₁, 242b₁ of each pawl 242a, 242b extends away from rack member 241 and is situated within an internal corner of respective V-shaped channel 232c, 232d of lower channel 230. The interface of the remote corner 242a₁, 242b₁ of each pawl 242a, 242b and respective V-shaped channel 232c, 232d of lower channel 230defines a pivot point for pawls 242a, 242b. A degree of pivoting of each pawl 242a, 242b is limited by the relative shape and dimension of pawls 242a, 242b and the relative angular orientation of respective V-shaped channel 232c, 232d of lower channel 230.

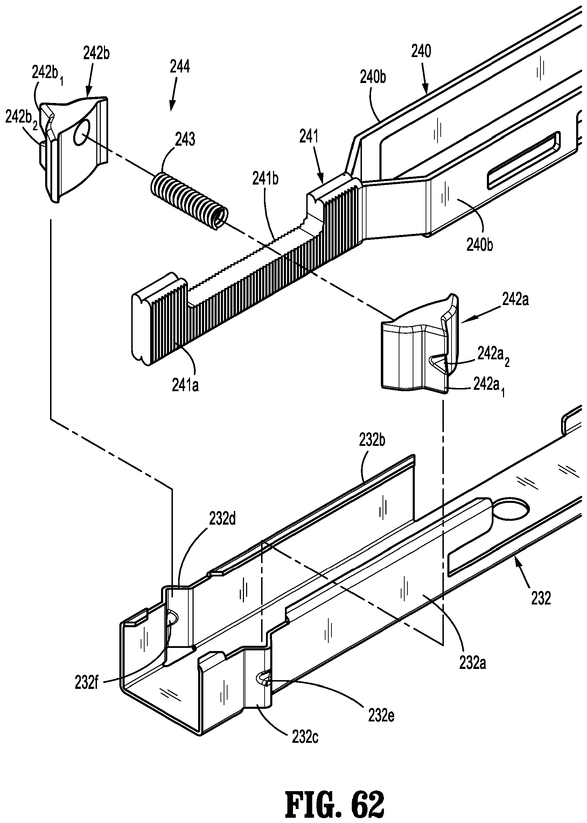

Additionally, as seen in FIG. 62, remote corner 242a₁, 242b₁ of each pawl 242a, 242b defines a respective notch 242a₂, 242b₂ formed therein. Notches 242a₂, 242b₂ of each pawl 242a, 242b are configured to receive respective ribs 232e, 232f of V-shaped channels 232c, 232d of lower channel 230 to as to maintain pawls 242a, 242b in axial registration with V-shaped channels 232c, 232d and rack member 241.

Similar to ratchet mechanism 144, ratchet mechanism 244 includes a snap-over spring 143, in the form of a coil spring, interposed between pawls 242a, 242b and extending over and across a slot defined in rack member 241. Snap-over spring 143 has a length sufficient to urge each pawl 242a, 242b into respective V-shaped channels 232c, 232d of lower channel 230.

Snap-over spring 143 functions to maintain the teeth of pawls 242a, 242b in engagement with the respective rack teeth 241a, 241b of rack member 241 as rack member 241 is axially translated. When pawls 242a, 242b are in a first position (permitting drive channel 240 to move in a distal direction), snap-over spring 243 bulges in a proximal direction, and when pawls 242a, 242b are in a second position (permitting drive channel 240 to move in a proximal direction), snap-over spring 143 bulges in a distal direction.

In operation, when drive channel 240 is moved to the distal-most position, a distally oriented surface of rack member 241 engages distally bulging snap-over spring 143 to reverse the direction of buckling thereof, and thus reverse or change the orientation of pawls 242a, 242b. Additionally, when drive channel 240 is moved to the proximal-most position, a proximally oriented surface of rack member 241 engages proximally bulging snap-over spring 143 to again reverse the direction of buckling thereof, and thus again reverse or change the orientation of pawls 242a, 242b.

Clip applier 100 may also include audible/tactile indication or feedback with lower channel 230 and pawls 242a, 242b, when pawls 242a, 242b are flipped at either end of the stroke of drive channel 240. In particular, when pawls 242a, 242b flip, pawls 242a, 242b are accelerated by snap-over spring 243 and slap against the walls of respective V-shaped channels 232c, 232d of lower channel 230, thereby providing the user feedback that end of a stroke has been reached.

Some clip appliers may experience premature lockout. For example, lockout features are often designed to provide lockout after all clips are fired. In certain instances, overstroke, such as pusher overstroke, can cause and/or contribute to premature lockout. There can be various causes for overstroke including undesirable momentum during handle closure and/or excessive friction generated between the drive channel and the pusher that causes the pusher to retract before moving forward when the handles are opened.

In order to limit and/or prevent premature lockout resulting from overstroke, embodiments of the presently described clip appliers can include one or more premature lockout prevention features such as those described herein below.

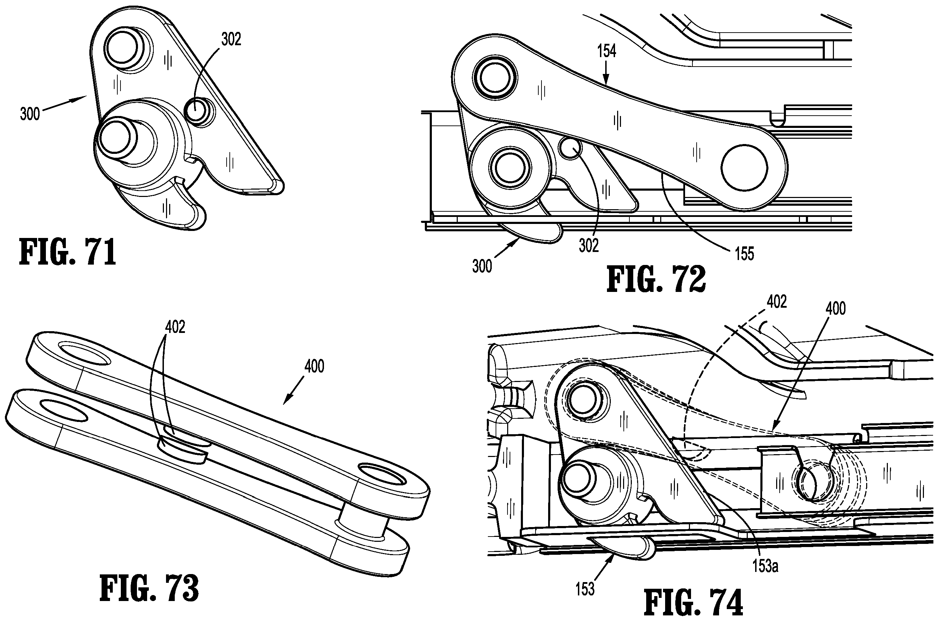

For example, as seen in FIGS. 71 and 72, one embodiment of a driver link 300 may include one or more stop members 302 extending therefrom. Although shown extending from an outer side surface of driver link 300, the one or more stop members 302 can be coupled to, and/or integrally formed with, any suitable surface of driver link 300. Each stop member 302 is configured to engage a first surface 155 of driven link 154 to provide a positive stop against first surface 155 of driven link 154 and prevent over rotation of driver link 300, thereby inhibiting and/or preventing premature lockout of clip applier 100.

As seen in FIGS. 73 and 74, one embodiment of a driven link 400 may include one or more stop members 402 extending therefrom. Although shown extending from an inner side surface of driven link 400, the one or more stop members 402 can be coupled to, and/or integrally formed with, any suitable surface of driven link 400. Similar to stop member(s) 302 of driver link 300, each stop member 402 of driven link 400 is configured to engage a first or distal surface 153a of driver link 152 to provide a positive stop against distal surface 153a of driver link 152 and prevent over rotation of driver link 152, thereby also inhibiting and/or preventing premature lockout of surgical clip applier 100.

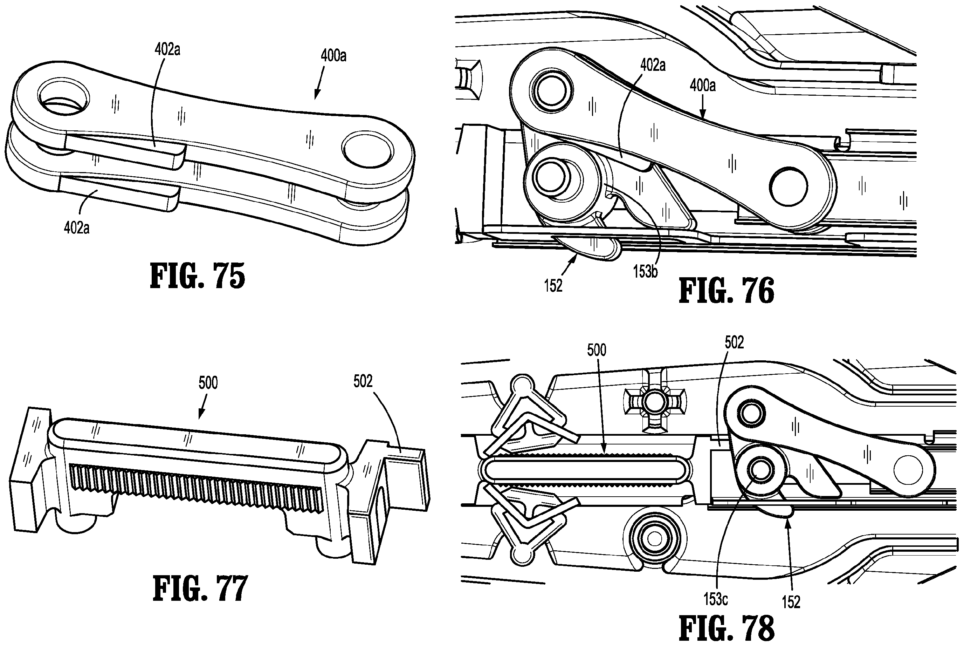

While stop member(s) 302 of driver link 300 is/are illustrated as rounded (see FIGS. 71 and 72), and stop member(s) 402 of driven link 400 is/are illustrated as semi-circular (see FIGS. 73 and 74), any of the presently described stop members can have any suitable shape and/or configuration. For example, with reference to FIGS. 75 and 76, another embodiment of a driven link 400a may include a stop member 402a. Stop member 402a may include a tapered and/or triangular configuration. Similar to stop member 402 of driven link 400, stop member 402a may be configured to engage a second or intermediate surface 153b of driver link 152 to provide a positive stop against intermediate surface 153b and prevent over rotation of driver link 152, thereby inhibiting and/or preventing premature lockout of clip applier 100.

As seen in FIGS. 77 and 78, one embodiment of a rack member 500 may include a distally extending stop member or post 502. Distally extending post 502 may be coupled to, and/or integrally formed with, rack member 500. Rack member 500 may have any suitable shape and/or dimension. A distal end of distally extending post 502 may be configured to engage a third or proximal surface 153c of driver link 152 to provide a positive stop against proximal surface 153c and prevent over rotation of driver link 152, thereby inhibiting and/or preventing premature lockout of clip applier 100.

It is contemplated that the shape and/or dimension of one or more of the presently disclosed stop members may be configured to provide a positive stop against one or more suitable surfaces of one or more components of any of the presently described clip appliers.

It should be understood that the foregoing description is only illustrative of the present disclosure. Various alternatives and modifications can be devised by those skilled in the art without departing from the disclosure.

For example, it is contemplated that coiled or spooled end 176c of constant force spring 176 may be attached or secured in clip applier 100 in such a way that coiled or spooled end 176c remains stationary near the pair of jaws 120 while the free end (i.e., 176b) of constant force spring 176 is attached to follower 174. In this embodiment, coiled or spooled end 176c of constant force spring 176 would not need to translate along the shaft of clip applier 100.

The embodiments described with reference to the attached drawing figures are presented only to demonstrate certain examples of the disclosure. The scope of the invention is defined in the appended claims

## Claims

1. A surgical clip applier (100), comprising:
a housing (104) comprising a pin (126);
at least one handle (106) movably connected to the housing (104);
a channel assembly (108) extending from the housing (104);
a clip carrier (170) disposed within the channel assembly (108) and defining a channel (170c);
a plurality of clips (C) loaded in the channel (170c) of the clip carrier (170);
a drive channel (140), operably connected to the at least one handle (106), movably supported in the housing (104) and the channel assembly (108), and comprising a tab (140f);
a clip pusher bar (160) reciprocally positioned within at least one of the housing (104) and the channel assembly (108); and
a linkage mechanism (150) that couples the drive channel (140) to the clip pusher bar (160), the linkage mechanism (150) comprising a driver link arm (152) pivotally connected to a driven link arm (154);
wherein the driven link arm (154) comprises a first, distal end (154a) pivotally connected to proximal end (160b) of pusher bar (160), the driver link arm (152) comprising a first, distal end (152a) pivotally connected to a second, proximal end (154b) of driven link arm (154), and a second, proximal end (152b) defining a slot (152c) configured to slidably receive the tab (140f) of drive channel (140), the driver link arm (152) defining a central eyelet (152c) for pivotal connection to the pin (126), such that driver link arm (152) is axially fixed with respect to housing (104);
wherein the surgical clip applier is configured such that actuating the at least one handle (106) transmits distal axial movement to the drive channel (140), causing the tab (140f) to engage the slot (152c), rotating driver link arm (152) about the pin (126), and pulling driven link arm (154) in a proximal direction which retracts the clip pusher bar (160) in a proximal direction, wherein returning the at least one handle (106) to an un-actuated position transmits proximal axial movement to the drive channel (140);
and **characterized in that** one or both of the driver link arm (152) and the driven link arm (154) comprises one or more stop members (302; 402) extending therefrom configured to limit over-stroke of the clip pusher bar (160) by providing a positive stop against the other of the driver link arm and the driven link arm, preventing over-rotation of the driver link arm (152).

2. A surgical clip applier (100), comprising:
a housing (104) comprising a pin (126);
at least one handle (106) movably connected to the housing (104);
a channel assembly (108) extending from the housing (104);
a clip carrier (170) disposed within the channel assembly (108) and defining a channel (170c);
a plurality of clips (C) loaded in the channel (170c) of the clip carrier (170);
a drive channel (140), operably connected to the at least one handle (106), movably supported in the housing (104) and the channel assembly (108), and comprising a tab (140f);
a clip pusher bar (160) reciprocally positioned within at least one of the housing (104) and the channel assembly (108); and
a linkage mechanism (150) that couples the drive channel (140) to the clip pusher bar (160), the linkage mechanism (150) comprising a driver link arm (152) pivotally connected to a driven link arm (154);
wherein the driven link arm (154) comprises a first, distal end (154a) pivotally connected to proximal end (160b) of pusher bar (160), the driver link arm (152) comprising a first, distal end (152a) pivotally connected to a second, proximal end (154b) of driven link arm (154), and a second, proximal end (152b) defining a slot (152c) configured to slidably receive the tab (140f) of drive channel (140), the driver link arm (152) defining a central eyelet (152c) for pivotal connection to the pin (126), such that driver link arm (152) is axially fixed with respect to housing (104);
wherein the surgical clip applier is configured such that actuating the at least one handle (106) transmits distal axial movement to the drive channel (140), causing the tab (140f) to engage the slot (152c), rotating driver link arm (152) about the pin (126), and pulling driven link arm (154) in a proximal direction which retracts the clip pusher bar (160) in a proximal direction, and wherein returning the at least one handle (106) to an un-actuated position transmits proximal axial movement to the drive channel (140);
further comprising a ratchet mechanism (144) disposed within the housing (104), the ratchet mechanism (144) including:
a rack member (500) associated with the drive channel (140);
a first pawl (142a) and a second pawl (142b) supported in the housing (104) adjacent to the rack member (141); and
a spring (143) interposed between the first pawl (142a) and the second pawl (142b), the spring (143) configured to enable movement of the rack member (500);
**characterized in that** the rack member (500) includes a distally extending stop member (502) configured to limit over-stroke of the clip pusher bar (160) by contacting the driver link arm (152), and provide a positive stop preventing over-rotation of the driver link arm (152).

3. The surgical clip applier (100) of claim 1, further comprising:
a ratchet mechanism (144) disposed within the housing (104), the ratchet mechanism (144) including:
a rack member (141) associated with the drive channel (140);
a first pawl (142a) and a second pawl (142b) supported in the housing (104) adjacent to the rack member (141); and
a spring (143) interposed between the first pawl (142a) and the second pawl (142b), the spring (143) configured to enable movement of the rack member (141).

4. The surgical clip applier (100) according to claim 2 or claim 3, further comprising:
a clip follower (174) slidably disposed within the channel (170c) of the clip carrier (170) and disposed proximally of the plurality of clips (C), the clip follower (174) configured for selective incremental advancement through the channel (170c) of the clip carrier (170) and through the channel assembly (108), wherein the clip follower (174) is configured to urge the plurality of clips (C), in a distal direction relative to the clip carrier (170), following a loading of a distal-most clip (C_{d}) of the plurality of clips (C) into a pair of jaws (120).

5. The surgical clip applier (100) according to claim 4, wherein the clip follower (174) is urged in a distal direction by a biasing member (176); preferably wherein the biasing member (176) is a constant force spring (176); preferably still wherein the constant force spring (176) is connected to the clip follower (174) and configured to move the clip follower (174) distally.

6. The surgical clip applier (100) according to claim 4, wherein the clip pusher bar (160) has a first end operatively connected to the at least one handle (106) and a second end defining a pusher (160c), the clip pusher bar (160) being movable away from the pair of jaws (120) as the at least one handle (106) is actuated in order to move the pusher (160c) behind the distal-most clip (C_{d}), and the clip pusher bar (160) configured to move towards the pair of jaws (120) as the at least one handle (106) is returned to an un-actuated position to move the distal-most clip (C_{d}) between the pair of jaws (120); preferably wherein the clip follower (174) includes a head configured for engagement by the pusher (160c) of the clip pusher bar (160), when in a retracted position, following a loading of a final clip (Cₚ) of the plurality of clips (C) into the pair of jaws (120) of the surgical clip applier (100).

7. The surgical clip applier (100) according to claim 6, wherein following engagement of the head of the clip follower (174) by the pusher (160c) of the clip pusher bar (160), a distal advancement of the clip pusher bar (160) will advance the clip follower (174) distally such that the head of the clip follower (174) is positioned between the pair of jaws (120); preferably wherein when the head of the clip follower (174) is positioned between the pair of jaws (120), the head of the clip follower (174) prevents the pair of jaws (120) from closing and the at least one handle (106) from actuating completely.

8. The surgical clip applier (100) according to claim 7, further comprising a jaw assembly (110) including the pair of jaws (120) extending from an end of the channel assembly (108), opposite the housing (104), the jaw assembly (110) adapted to accommodate a clip (C) therein and operable to effectuate formation of a clip (C) in response to movement of the at least one handle (106).

9. The surgical clip applier (100) according to claim 8, wherein the drive channel (140) includes a first end operatively connected to the at least one handle (106) and a second end configured to surround and selectively engage the pair of jaws (120) to effectuate closure of the pair of jaws (120);
the drive channel (140) movable towards the jaw assembly (110) as the at least one handle (106) moves in a first direction to move the second end of the drive channel (140) against the pair of jaws (120) to close the pair of jaws (120);
the drive channel (140) movable away from the jaw assembly (110) as the at least one handle (106) moves in a second direction, opposite the first direction, to move the second end of the drive channel (140) away from the jaw assembly (110) and enable the pair of jaws (120) to open.

10. The surgical clip applier (100) according to claim 9, wherein, with the drive channel (140) disposed at a proximal-most position, with the spring (143) bowed in a proximal direction, and with the first pawl (142a) and the second pawl (142b) engaged with the rack member (141) so as to enable the drive channel (140) to move in the distal direction, while the drive channel (140) is advanced to a distal-most position, the spring (143), while proximally bowing, is configured to bow in the distal direction, whereby the drive channel (140) moves in the proximal direction.

11. The surgical clip applier (100) according to claim 9, wherein, with the drive channel (140) disposed at the distal-most position, with the spring (143) bowed in the distal direction, and with the first pawl (142a) and the second pawl (142b) engaged with the rack member (141) so as to enable the drive channel (140) to move in the proximal direction, while the drive channel (140) is retracted to the proximal-most position, the spring (143), while distally bowing, is configured to bow in the proximal direction, whereby the drive channel (140) moves in the distal direction.

12. The surgical clip applier (100) according to claim 9, wherein the drive channel (140) includes a stop tab (140g) projecting toward the clip follower (174), and wherein the clip follower (174) defines a window (174d₁) therein,
wherein, while the clip follower (174) is in a distal-most position with the head thereof disposed between the pair of jaws (120), and while the drive channel (140) is in the proximal-most position, the stop tab (140g) of the drive channel (140) is disposed in the window (174d₁) of the clip follower (174).

13. The surgical clip applier (100) according to claim 12, wherein the clip follower (174) includes:
an elongate body (174a) having a distal end and a proximal end, wherein the head is supported at the distal end thereof; and
a tail (174d) having a distal end and a proximal end, wherein the proximal end of the tail (174d) is connected to the proximal end of the elongate body (174a) such that the tail (174d) and the elongate body (174a) bias away from one another.

14. The surgical clip applier (100) according to claim 12, wherein the window (174d₁) of the clip follower (174), configured to receive the stop tab (140g) of the drive channel (140), is formed in the tail (174d).

15. The surgical clip applier (100) according to claim 14, wherein the biasing member (176) is a constant force spring (176), the constant force spring (176) including a distal end (176b) secured against movement in the surgical clip applier, and a proximal end (176a) coiled onto itself and at least partially disposed in the window (174d₁) of the tail (174d) of the clip follower (174), wherein the coiled proximal end (176a) of the constant force spring (176) draws the clip follower (174) distally upon a coiling of the coiled proximal end (176a) of the constant force spring (176) subsequent to a loading of a distal-most clip (C_{d}) of the plurality of clips (C) into the pair of jaws (120) of the surgical clip applier (100).

## Patentansprüche

1. Chirurgische Klammeranbringvorrichtung (100), umfassend:
ein Gehäuse (104), umfassend einen Stift (126);
mindestens einen Griff (106), der beweglich mit dem Gehäuse (104) verbunden ist;
eine Kanalanordnung (108), die sich vom Gehäuse (104) aus erstreckt;
einen Klammerträger (170), der innerhalb der Kanalanordnung (108) angeordnet ist und einen Kanal (170c) definiert;
mehrere in den Kanal (170c) des Klammerträgers (170) geladene Klammern (C);
einen Antriebskanal (140), der betriebsmäßig mit dem mindestens einen Griff (106) verbunden ist, der beweglich in dem Gehäuse (104) und der Kanalanordnung (108) gelagert ist und der eine Nase (140f) umfasst;
eine Klammer-Drückerstange (160), die wechselseitig innerhalb des Gehäuses (104) und/oder der Kanalanordnung (108) angeordnet ist; und
einen Gestängemechanismus (150), der den Antriebskanal (140) mit der Klammer-Drückerstange (160) koppelt, wobei der Gestängemechanismus (150) einen Antriebsgestängearm (152) umfasst, der schwenkbar mit einem angetriebenen Gestängearm (154) verbunden ist;
wobei der angetriebene Gestängearm (154) ein erstes, distales Ende (154a) umfasst, das schwenkbar mit dem proximalen Ende (160b) der Drückerstange (160) verbunden ist, wobei der Antriebsgestängearm (152) ein erstes, distales Ende (152a) umfasst, das schwenkbar mit einem zweiten, proximalen Ende (154b) des angetriebenen Gestängearms (154) verbunden ist, und ein zweites, proximales Ende (152b), das einen Schlitz (152c) definiert, der so konfiguriert ist, dass er die Nase (140f) des Antriebskanals (140) gleitend aufnimmt, wobei der Antriebsgestängearm (152) eine zentrale Öse (152c) zur schwenkbaren Verbindung mit dem Stift (126) definiert, derart, dass der Antriebsgestängearm (152) in Bezug auf das Gehäuse (104) axial fixiert ist;
wobei die chirurgische Klammeranbringungsvorrichtung derart konfiguriert ist, dass die Betätigung des mindestens einen Griffs (106) eine distale axiale Bewegung auf den Antriebskanal (140) überträgt, wodurch die Nase (140f) in den Schlitz (152c) eingreift, wobei der Antriebsgestängearm (152) um den Stift (126) gedreht wird, und einen angetriebenen Gestängearm (154) in eine proximale Richtung zieht, der die Klammer-Drückerstange (160) in einer proximalen Richtung zurückzieht, wobei das Zurückführen des mindestens einen Griffs (106) in eine unbetätigte Position eine proximale axiale Bewegung auf den Antriebskanal (140) überträgt;
und **dadurch gekennzeichnet, dass** entweder der Antriebsgestängearm (152) oder der angetriebene Gestängearm (154) oder beide ein oder mehrere sich davon erstreckende Anschlagelemente (302; 402) umfassen, die so konfiguriert sind, dass sie einen Überhub der Klammer-Drückerstange (160) begrenzen, indem sie einen positiven Anschlag gegen den jeweils anderen, d. h. den Antriebsgestängearm oder den angetriebenen Gestängearm, bereitstellen, wodurch ein Überdrehen des Antriebsgestängearms (152) verhindert wird.

2. Chirurgische Klammeranbringvorrichtung (100), umfassend:
ein Gehäuse (104), umfassend einen Stift (126);
mindestens einen Griff (106), der beweglich mit dem Gehäuse (104) verbunden ist;
eine Kanalanordnung (108), die sich vom Gehäuse (104) aus erstreckt;
einen Klammerträger (170), der innerhalb der Kanalanordnung (108) angeordnet ist und einen Kanal (170c) definiert;
mehrere in den Kanal (170c) des Klammerträgers (170) geladene Klammern (C);
einen Antriebskanal (140), der betriebsmäßig mit dem mindestens einen Griff (106) verbunden ist, der beweglich in dem Gehäuse (104) und der Kanalanordnung (108) gelagert ist und der eine Nase (140f) umfasst;
eine Klammer-Drückerstange (160), die wechselseitig innerhalb des Gehäuses (104) und/oder der Kanalanordnung (108) angeordnet ist; und
einen Gestängemechanismus (150), der den Antriebskanal (140) mit der Klammer-Drückerstange (160) koppelt, wobei der Gestängemechanismus (150) einen Antriebsgestängearm (152) umfasst, der schwenkbar mit einem angetriebenen Gestängearm (154) verbunden ist;
wobei der angetriebene Gestängearm (154) ein erstes, distales Ende (154a) umfasst, das schwenkbar mit dem proximalen Ende (160b) der Drückerstange (160) verbunden ist, wobei der Antriebsgestängearm (152) ein erstes, distales Ende (152a) umfasst, das schwenkbar mit einem zweiten, proximalen Ende (154b) des angetriebenen Gestängearms (154) verbunden ist, und ein zweites, proximales Ende (152b), das einen Schlitz (152c) definiert, der so konfiguriert ist, dass er die Nase (140f) des Antriebskanals (140) gleitend aufnimmt, wobei der Antriebsgestängearm (152) eine zentrale Öse (152c) zur schwenkbaren Verbindung mit dem Stift (126) definiert, derart, dass der Antriebsgestängearm (152) in Bezug auf das Gehäuse (104) axial fixiert ist;
wobei die chirurgische Klammeranbringungsvorrichtung derart konfiguriert ist, dass die Betätigung des mindestens einen Griffs (106) eine distale axiale Bewegung auf den Antriebskanal (140) überträgt, wodurch die Nase (140f) in den Schlitz (152c) eingreift, wobei der Antriebsgestängearm (152) um den Stift (126) gedreht wird, und einen angetriebenen Gestängearm (154) in eine proximale Richtung zieht, der die Klammer-Drückerstange (160) in einer proximalen Richtung zurückzieht, und wobei das Zurückführen des mindestens einen Griffs (106) in eine unbetätigte Position eine proximale axiale Bewegung auf den Antriebskanal (140) überträgt;
ferner umfassend einen Ratschenmechanismus (144), der innerhalb des Gehäuses (104) angeordnet ist, wobei der Ratschenmechanismus (144) Folgendes umfasst:
ein Zahnstangenelement (500), das mit dem Antriebskanal (140) gekoppelt ist;
eine erste Sperrklinke (142a) und eine zweite Sperrklinke (142b), die in dem Gehäuse (104) neben dem Zahnstangenelement (141) gelagert sind; und
eine Feder (143), die zwischen der ersten Sperrklinke (142a) und der zweiten Sperrklinke (142b) angeordnet ist, wobei die Feder (143) so konfiguriert ist, dass sie die Bewegung des Zahnstangenelements (500) ermöglicht;
**dadurch gekennzeichnet, dass** das Zahnstangenelement (500) ein sich distal erstreckendes Anschlagelement (502) umfasst, das so konfiguriert ist, dass es einen Überhub der Klammer-Drückerstange (160) durch Kontakt mit dem Antriebsgestängearm (152) begrenzt und einen positiven Anschlag bereitstellt, der ein Überdrehen des Antriebsgestängearms (152) verhindert.

3. Chirurgische Klammeranbringvorrichtung (100) nach Anspruch 1, ferner umfassend:
einen Ratschenmechanismus (144), der innerhalb des Gehäuses (104) angeordnet ist, wobei der Ratschenmechanismus (144) Folgendes umfasst:
ein Zahnstangenelement (141), das mit dem Antriebskanal (140) gekoppelt ist;
eine erste Sperrklinke (142a) und eine zweite Sperrklinke (142b), die in dem Gehäuse (104) neben dem Zahnstangenelement (141) gelagert sind; und
eine Feder (143), die zwischen der ersten Sperrklinke (142a) und der zweiten Sperrklinke (142b) angeordnet ist, wobei die Feder (143) so konfiguriert ist, dass sie die Bewegung des Zahnstangenelements (141) ermöglicht.

4. Chirurgische Klammeranbringvorrichtung (100) nach Anspruch 2 oder Anspruch 3, ferner umfassend:
einen Klammernachläufer (174), der verschiebbar innerhalb des Kanals (170c) des Klammerträgers (170) angeordnet ist und proximal zu den mehreren Klammern (C) angeordnet ist, wobei der Klammernachläufer (174) für ein selektives schrittweises Vorrücken durch den Kanal (170c) des Klammerträgers (170) und durch die Kanalanordnung (108) konfiguriert ist, wobei der Klammernachläufer (174) so konfiguriert ist, dass er die mehreren Klammern (C) nach dem Laden einer am weitesten distal gelegenen Klammer (C_{d}) der mehreren Klammern (C) in ein Backenpaar (120) in eine distale Richtung relativ zu dem Klammerträger (170) drückt.

5. Chirurgische Klammeranbringvorrichtung (100) nach Anspruch 4, wobei der Klammernachläufer (174) durch ein Vorspannelement (176) in eine distale Richtung gedrängt wird;
wobei vorzugsweise das Vorspannelement (176) eine Konstantkraftfeder (176) ist;
wobei vorzugsweise ferner die Konstantkraftfeder (176) mit dem Klammernachläufer (174) verbunden ist und so konfiguriert ist, dass sie den Klammernachläufer (174) in distaler Richtung bewegt.

6. Chirurgische Klammeranbringvorrichtung (100) nach Anspruch 4, wobei die Klammer-Drückerstange (160) ein erstes Ende aufweist, das operativ mit dem mindestens einen Griff (106) verbunden ist, und ein zweites Ende, das einen Drücker (160c) definiert, wobei die Klammer-Drückerstange (160) von dem Backenpaar (120) weg bewegbar ist, wenn der mindestens eine Griff (106) betätigt wird, um den Drücker (160c) hinter die am weitesten distal gelegene Klammer (C_{d}) zu bewegen, und wobei die Klammer-Drückerstange (160) so konfiguriert ist, dass sie sich auf das Backenpaar (120) zu bewegt, wenn der mindestens eine Griff (106) in eine unbetätigte Position zurückgebracht wird, um die am weitesten distal gelegene Klammer (C_{d}) zwischen dem Backenpaar (120) zu bewegen;
wobei vorzugsweise der Klammernachläufer (174) einen Kopf aufweist, der so konfiguriert ist, dass der Drücker (160c) der Klammer-Drückerstange (160), wenn er sich in einer zurückgezogenen Position befindet, nach dem Laden einer letzten Klammer (Cp) der mehreren Klammern (C) in das Backenpaar (120) der chirurgischen Klammeranbringvorrichtung (100) eingreift.

7. Chirurgische Klammeranbringvorrichtung (100) nach Anspruch 6, wobei nach dem Eingreifen des Kopfes des Klammernachläufers (174) durch den Drücker (160c) der Klammer-Drückerstange (160) eine distale Vorwärtsbewegung der Klammer-Drückerstange (160) den Klammernachläufer (174) nach distal vorwärts bewegt, derart, dass der Kopf des Klammernachläufers (174) zwischen dem Backenpaar (120) positioniert wird;
wobei vorzugsweise dann, wenn der Kopf des Klammernachläufers (174) zwischen dem Backenpaar (120) positioniert ist, der Kopf des Klammernachläufers (174) verhindert, dass sich das Backenpaar (120) schließt und der mindestens eine Griff (106) vollständig betätigt wird.

8. Chirurgische Klammeranbringvorrichtung (100) nach Anspruch 7, ferner umfassend eine Backenanordnung (110), die das Backenpaar (120) umfasst, das sich von einem Ende der Kanalanordnung (108) gegenüber dem Gehäuse (104) erstreckt, wobei die Backenanordnung (110) so angepasst ist, dass sie eine Klammer (C) darin aufnehmen kann, und betätigt werden kann, um die Bildung einer Klammer (C) als Reaktion auf die Bewegung des mindestens einen Griffs (106) zu bewirken.

9. Chirurgische Klammeranbringvorrichtung (100) nach Anspruch 8, wobei der Antriebskanal (140) ein erstes Ende, das operativ mit dem mindestens einen Griff (106) verbunden ist, und ein zweites Ende umfasst, das so konfiguriert ist, dass es das Backenpaar (120) umgibt und selektiv in dieses eingreift, um ein Schließen des Backenpaares (120) zu bewirken;
wobei der Antriebskanal (140) in Richtung der Backenanordnung (110) bewegbar ist, wenn sich der mindestens eine Griff (106) in eine erste Richtung bewegt, um das zweite Ende des Antriebskanals (140) gegen das Backenpaar (120) zu bewegen, um das Backenpaar (120) zu schließen;
wobei der Antriebskanal (140) von der Backenanordnung (110) weg bewegbar ist, wenn sich der mindestens eine Griff (106) in eine zweite Richtung, entgegengesetzt zur ersten Richtung, bewegt, um das zweite Ende des Antriebskanals (140) von der Backenanordnung (110) weg zu bewegen und das Öffnen des Backenpaares (120) zu ermöglichen.

10. Chirurgische Klammeranbringvorrichtung (100) nach Anspruch 9, wobei der Antriebskanal (140) in der proximalsten Position angeordnet ist, wobei die Feder (143) in proximaler Richtung gebogen ist und wobei die erste Sperrklinke (142a) und die zweite Sperrklinke (142b) mit dem Zahnstangenelement (141) in Eingriff stehen, so dass sich der Antriebskanal (140) in distaler Richtung bewegen kann, wobei die Feder (143), während der Antriebskanal (140) zu einer am weitesten distal gelegenen Position vorgeschoben wird, während sie sich proximal biegt, so konfiguriert ist, dass sie sich in die distale Richtung biegt, wodurch sich der Antriebskanal (140) in die proximale Richtung bewegt.

11. Chirurgische Klammeranbringvorrichtung (100) nach Anspruch 9, wobei der Antriebskanal (140) in der am weitesten distal gelegenen Position angeordnet ist, wobei die Feder (143) in distaler Richtung gebogen ist, und wobei die erste Sperrklinke (142a) und die zweite Sperrklinke (142b) mit dem Zahnstangenelement (141) in Eingriff stehen, so dass sich der Antriebskanal (140) in proximaler Richtung bewegen kann, wobei, während der Antriebskanal (140) in die proximalste Position zurückgezogen ist, die Feder (143), während sie sich distal biegt, so konfiguriert ist, dass sie sich in proximaler Richtung biegt, wodurch sich der Antriebskanal (140) in distaler Richtung bewegt.

12. Chirurgische Klammeranbringvorrichtung (100) nach Anspruch 9, wobei der Antriebskanal (140) eine Anschlagnase (140g) aufweist, die in Richtung des Klammernachläufers (174) vorsteht, und wobei der Klammernachläufer (174) ein Fenster (174d₁) darin definiert, wobei, während sich der Klammernachläufer (174) in einer am weitesten distal gelegenen Position befindet, wobei sein Kopf zwischen dem Backenpaar (120) angeordnet ist, und wobei, während sich der Antriebskanal (140) in der am weitesten proximal gelegenen Position befindet, die Anschlagnase (140g) des Antriebskanals (140) im Fenster (174d₁) des Klammernachläufers (174) angeordnet ist.

13. Chirurgische Klammeranbringvorrichtung (100) nach Anspruch 12, wobei der Klammernachläufer (174) Folgendes umfasst:
einem länglichen Körper (174a) mit einem distalen Ende und einem proximalen Ende, wobei der Kopf an seinem distalen Ende gehalten wird; und
ein Endstück (174d) mit einem distalen Ende und einem proximalen Ende, wobei das proximale Ende des Endstücks (174d) mit dem proximalen Ende des länglichen Körpers (174a) derart verbunden ist, dass das Endstück (174d) und der längliche Körper (174a) voneinander weg vorgespannt sind.

14. Chirurgische Klammeranbringvorrichtung (100) nach Anspruch 12, wobei das Fenster (174d₁) des Klammernachläufers (174), das so konfiguriert ist, dass es die Anschlagnase (140g) des Antriebskanals (140) aufnimmt, im Endstück (174d) ausgebildet ist.

15. Chirurgische Klammeranbringvorrichtung (100) nach Anspruch 14, wobei das Vorspannelement (176) eine Konstantkraftfeder (176) ist, wobei die Konstantkraftfeder (176) ein distales Ende (176b), das gegen Bewegungen in der chirurgischen Klammeranbringvorrichtung gesichert ist, und ein proximales Ende (176a) aufweist, das auf sich selbst gewickelt und zumindest teilweise im Fenster (174d₁) des Endstücks (174d) des Klammernachläufers (174) angeordnet ist, wobei das aufgewickelte proximale Ende (176a) der Konstantkraftfeder (176) den Klammernachläufer (174) nach einem Aufwickeln des aufgewickelten proximalen Endes (176a) der Konstantkraftfeder (176) in distaler Richtung zieht, nachdem eine am weitesten distal gelegene Klammer (C_{d}) der mehreren Klammern (C) in das Backenpaar (120) der chirurgischen Klammeranbringvorrichtung (100) geladen wurde.

## Revendications

1. Applicateur d'agrafes chirurgicales (100), comprenant :
un boîtier (104) comprenant une broche (126) ;
au moins une poignée (106) reliée de manière mobile au boîtier (104) ;
un ensemble de canal (108) s'étendant à partir du boîtier (104) ;
un support d'agrafes (170) disposé à l'intérieur de l'ensemble de canal (108) et définissant un canal (170c) ;
une pluralité d'agrafes (C) chargées dans le canal (170c) du support d'agrafes (170) ;
un canal d'entraînement (140), relié de manière fonctionnelle à l'au moins une poignée (106), supporté de manière mobile dans le boîtier (104) et l'ensemble de canal (108), et comprenant une languette (140f) ;
une barre de poussée d'agrafes (160) positionnée à titre de réciprocité dans le boîtier (104) et/ou l'ensemble de canal (108) ; et
un mécanisme de liaison (150) qui accouple le canal d'entraînement (140) à la barre de poussée d'agrafes (160), le mécanisme de liaison (150) comprenant un bras de liaison d'entraînement (152) relié de manière pivotante à un bras de liaison entraîné (154) ;
dans lequel le bras de liaison entraîné (154) comprend une première extrémité distale (154a) reliée de manière pivotante à l'extrémité proximale (160b) de la barre de poussée (160), le bras de liaison d'entraînement (152) comprenant une première extrémité distale (152a) reliée de manière pivotante à une seconde extrémité proximale (154b) du bras de liaison entraîné (154), et à une seconde extrémité proximale (152b) définissant une fente (152c) conçue pour recevoir de manière coulissante la languette (140f) du canal d'entraînement (140), le bras de liaison d'entraînement (152) définissant un œillet central (152c) pour une liaison pivotante à la broche (126), de telle sorte que le bras de liaison d'entraînement (152) est fixé axialement par rapport au boîtier (104) ;
dans lequel l'applicateur d'agrafes chirurgicales est conçu de telle sorte que l'actionnement de l'au moins une poignée (106) transmet un mouvement axial distal au canal d'entraînement (140), amenant la languette (140f) à mettre en prise la fente (152c), faisant pivoter le bras de liaison d'entraînement (152) autour de la broche (126), et tirant le bras de liaison entraîné (154) dans une direction proximale qui rétracte la barre de poussée d'agrafes (160) dans une direction proximale, dans lequel le retour de l'au moins une poignée (106) dans une position non actionnée transmet un mouvement axial proximal au canal d'entraînement (140) ;
et **caractérisé en ce que** l'un ou les deux bras de liaison d'entraînement (152) et le bras de liaison entraîné (154) comprennent un ou plusieurs éléments de butée (302; 402) s'étendant à partir de ceux-ci, configurés pour limiter la course excessive de la barre de poussée d'agrafes (160) en fournissant une butée fixe contre l'autre du bras de liaison d'entraînement et le bras de liaison entraîné, empêchant la rotation excessive du bras de liaison d'entraînement (152).

2. Applicateur d'agrafes chirurgicales (100), comprenant :
un boîtier (104) comprenant une broche (126) ;
au moins une poignée (106) reliée de manière mobile au boîtier (104) ;
un ensemble de canal (108) s'étendant à partir du boîtier (104) ;
un support d'agrafes (170) disposé à l'intérieur de l'ensemble de canal (108) et définissant un canal (170c) ;
une pluralité d'agrafes (C) chargées dans le canal (170c) du support d'agrafes (170) ;
un canal d'entraînement (140), relié de manière fonctionnelle à l'au moins une poignée (106), supporté de manière mobile dans le boîtier (104) et l'ensemble de canal (108), et comprenant une languette (140f) ;
une barre de poussée d'agrafes (160) positionnée à titre de réciprocité dans le boîtier (104) et/ou l'ensemble de canal (108) ; et
un mécanisme de liaison (150) qui accouple le canal d'entraînement (140) à la barre de poussée d'agrafes (160), le mécanisme de liaison (150) comprenant un bras de liaison d'entraînement (152) relié de manière pivotante à un bras de liaison entraîné (154) ;
dans lequel le bras de liaison entraîné (154) comprend une première extrémité distale (154a) reliée de manière pivotante à l'extrémité proximale (160b) de la barre de poussée (160), le bras de liaison d'entraînement (152) comprenant une première extrémité distale (152a) reliée de manière pivotante à une seconde extrémité proximale (154b) du bras de liaison entraîné (154), et à une seconde extrémité proximale (152b) définissant une fente (152c) conçue pour recevoir de manière coulissante la languette (140f) du canal d'entraînement (140), le bras de liaison d'entraînement (152) définissant un œillet central (152c) pour une liaison pivotante à la broche (126), de telle sorte que le bras de liaison d'entraînement (152) est fixé axialement par rapport au boîtier (104) ;
dans lequel l'applicateur d'agrafes chirurgicales est conçu de telle sorte que l'actionnement de l'au moins une poignée (106) transmet un mouvement axial distal au canal d'entraînement (140), amenant la languette (140f) à mettre en prise la fente (152c), faisant pivoter le bras de liaison d'entraînement (152) autour de la broche (126), et tirant le bras de liaison entraîné (154) dans une direction proximale qui rétracte la barre de poussée d'agrafes (160) dans une direction proximale, et dans lequel le retour de l'au moins une poignée (106) vers une position non actionnée transmet un mouvement axial proximal au canal d'entraînement (140) ;
comprenant en outre un mécanisme d'encliquetage (144) disposé à l'intérieur du boîtier (104), le mécanisme d'encliquetage (144) comportant :
un élément de crémaillère (500) associé au canal d'entraînement (140) ;
un premier cliquet (142a) et un second cliquet (142b) supportés dans le boîtier (104) adjacent à l'élément de crémaillère (141) ; et
un ressort (143) interposé entre le premier cliquet (142a) et le second cliquet (142b), le ressort (143) étant configuré pour permettre le mouvement de l'élément de crémaillère (500) ;
**caractérisé en ce que** l'élément de crémaillère (500) comporte un élément de butée s'étendant de manière distale (502) configuré pour limiter la course excessive de la barre de poussée d'agrafes (160) en mettant en contact le bras de liaison d'entraînement (152), et fournit une butée fixe empêchant la rotation excessive du bras de liaison d'entraînement (152).

3. Applicateur d'agrafes chirurgicales (100) selon la revendication 1, comprenant en outre
un mécanisme d'encliquetage (144) disposé à l'intérieur du boîtier (104), le mécanisme d'encliquetage (144) comprenant :
un élément de crémaillère (141) associé au canal d'entraînement (140) ;
un premier cliquet (142a) et un second cliquet (142b) supportés dans le boîtier (104) adjacent à l'élément de crémaillère (141) ; et
un ressort (143) interposé entre le premier cliquet (142a) et le second cliquet (142b), le ressort (143) étant configuré pour permettre le mouvement de l'élément de crémaillère (141).

4. Applicateur d'agrafes chirurgicales (100) selon la revendication 2 ou la revendication 3, comprenant en outre :
un prolongateur d'agrafes (174) disposé de manière coulissante à l'intérieur du canal (170c) du support d'agrafes (170) et disposé à proximité de la pluralité d'agrafes (C), le prolongateur d'agrafes (174) étant configuré pour un avancement incrémental sélectif à travers le canal (170c) du support d'agrafes (170) et à travers l'ensemble de canal (108), dans lequel le prolongateur d'agrafes (174) est conçu pour pousser la pluralité d'agrafes (C), dans une direction distale par rapport au support d'agrafes (170), suivant un chargement d'une agrafe la plus distale (C_{d}) de la pluralité d'agrafes (C) dans une paire de mâchoires (120).

5. Applicateur d'agrafes chirurgicales (100) selon la revendication 4, dans lequel le prolongateur d'agrafes (174) est poussé dans une direction distale par un élément de sollicitation (176) ;
de préférence dans lequel l'élément de sollicitation (176) est un ressort à force constante (176) ;
de préférence encore dans lequel le ressort à force constante (176) est relié au prolongateur d'agrafes (174) et configuré pour déplacer le prolongateur d'agrafes (174) de manière distale.

6. Applicateur d'agrafes chirurgicales (100) selon la revendication 4, dans lequel la barre de poussée d'agrafes (160) a une première extrémité reliée de manière fonctionnelle à l'au moins une poignée (106) et une seconde extrémité définissant un poussoir (160c), la barre de poussée d'agrafes (160) pouvant s'éloigner de la paire de mâchoires (120) lorsque l'au moins une poignée (106) est actionnée afin de déplacer le poussoir (160c) derrière l'agrafe la plus distale (C_{d}), et la barre de poussée d'agrafes (160) étant configurée pour se déplacer vers la paire de mâchoires (120) lorsque l'au moins une poignée (106) est ramenée dans une position non actionnée pour déplacer l'agrafe la plus distale (C_{d}) entre la paire de mâchoires (120) ;
de préférence dans lequel le prolongateur d'agrafes (174) comporte une tête configurée pour la mise en prise par le poussoir (160c) de la barre de poussée d'agrafes (160), lorsqu'il est en position rétractée, suivant un chargement d'un agrafe finale (Cₚ) de la pluralité d'agrafes (C) dans la paire de mâchoires (120) de l'applicateur d'agrafes chirurgicales (100).

7. Applicateur d'agrafes chirurgicales (100) selon la revendication 6, dans lequel suivant la mise en prise de la tête du prolongateur d'agrafes (174) par le poussoir (160c) de la barre de poussée d'agrafes (160), un avancement distal de la barre de poussée d'agrafes (160) fera avancer le prolongateur d'agrafes (174) de manière distale de telle sorte que la tête du prolongateur d'agrafes (174) soit positionnée entre la paire de mâchoires (120) ;
de préférence dans lequel lorsque la tête du prolongateur d'agrafes (174) est positionnée entre la paire de mâchoires (120), la tête du prolongateur d'agrafes (174) empêche la paire de mâchoires (120) de se fermer et l'au moins une poignée (106) de s'actionner complètement.

8. Applicateur d'agrafes chirurgicales (100) selon la revendication 7, comprenant en outre un ensemble de mâchoires (110) comportant la paire de mâchoires (120) s'étendant depuis une extrémité de l'ensemble de canal (108), opposée au boîtier (104), l'ensemble de mâchoires (110) étant adapté pour recevoir une agrafe (C) à l'intérieur de celui-ci et pouvant fonctionner pour effectuer la formation d'une agrafe (C) en réponse au mouvement de l'au moins une poignée (106).

9. Applicateur d'agrafes chirurgicales (100) selon la revendication 8, dans lequel le canal d'entraînement (140) comporte une première extrémité reliée de manière fonctionnelle à l'au moins une poignée (106) et une seconde extrémité configurée pour entourer et mettre en prise sélectivement la paire de mâchoires (120) afin d'effectuer la fermeture de la paire de mâchoires (120) ;
le canal d'entraînement (140) mobile vers l'ensemble de mâchoires (110) lorsque l'au moins une poignée (106) se déplace dans une première direction pour déplacer la seconde extrémité du canal d'entraînement (140) contre la paire de mâchoires (120) afin de fermer la paire de mâchoires (120) ;
le canal d'entraînement (140) pouvant être éloigné de l'ensemble de mâchoires (110) lorsque l'au moins une poignée (106) se déplace dans une seconde direction, opposée à la première direction, pour déplacer la seconde extrémité du canal d'entraînement (140) de l'ensemble de mâchoires (110) et permettre à la paire de mâchoires (120) de s'ouvrir.

10. Applicateur d'agrafes chirurgicales (100) selon la revendication 9, dans lequel, avec le canal d'entraînement (140) disposé à une position la plus proximale, avec le ressort (143) courbé dans une direction proximale, et avec le premier cliquet (142a) et le second cliquet (142b) en prise avec l'élément de crémaillère (141) de manière à permettre au canal d'entraînement (140) de se déplacer dans la direction distale, tandis que le canal d'entraînement (140) est avancé vers une position la plus distale, le ressort (143), alors qu'il s'incline de manière proximale, est configuré pour s'incliner dans la direction distale, moyennant quoi le canal d'entraînement (140) se déplace dans la direction proximale.

11. Applicateur d'agrafes chirurgicales (100) selon la revendication 9, le canal d'entraînement (140) étant disposé dans la position la plus distale, le ressort (143) étant courbé dans la direction distale, et le premier cliquet (142a) et le second cliquet (142b) étant en prise avec l'élément de crémaillère (141) de manière à permettre au canal d'entraînement (140) de se déplacer dans la direction proximale, tandis que le canal d'entraînement (140) est rétracté vers la position la plus proximale, le ressort (143), tandis que s'inclinant de manière distale, est configuré pour s'incliner dans la direction proximale, moyennant quoi le canal d'entraînement (140) se déplace dans la direction distale.

12. Applicateur d'agrafes chirurgicales (100) selon la revendication 9, dans lequel le canal d'entraînement (140) comporte une languette de butée (140g) faisant saillie vers le prolongateur d'agrafes (174), et dans lequel le prolongateur d'agrafes (174) définit une fenêtre (174d₁) dans celui-ci, dans lequel, tandis que le prolongateur d'agrafes (174) est dans une position la plus distale avec sa tête disposée entre la paire de mâchoires (120), et tandis que le canal d'entraînement (140) est dans la position la plus proximale, la languette de butée (140g) du canal d'entraînement (140) est disposée dans la fenêtre (174d₁) du prolongateur d'agrafes (174).

13. Applicateur d'agrafes chirurgicales (100) selon la revendication 12, dans lequel le prolongateur d'agrafes (174) comporte :
un corps allongé (174a) ayant une extrémité distale et une extrémité proximale, dans lequel la tête est supportée à son extrémité distale ; et
une queue (174d) ayant une extrémité distale et une extrémité proximale, l'extrémité proximale de la queue (174d) étant reliée à l'extrémité proximale du corps allongé (174a) de telle sorte que la queue (174d) et le corps allongé (174a) s'écartent l'un de l'autre.

14. Applicateur d'agrafes chirurgicales (100) selon la revendication 12, dans lequel la fenêtre (174d₁) du prolongateur d'agrafes (174), étant configurée pour recevoir la languette de butée (140g) du canal d'entraînement (140), est formée dans la queue (174d).

15. Applicateur d'agrafes chirurgicales (100) selon la revendication 14, dans lequel l'élément de sollicitation (176) est un ressort à force constante (176), le ressort à force constante (176) comprenant une extrémité distale (176b) fixée contre le mouvement dans l'applicateur d'agrafes chirurgicales, et une extrémité proximale (176a) enroulée sur elle-même et au moins partiellement disposée dans la fenêtre (174d₁) de la queue (174d) du prolongateur d'agrafes (174), l'extrémité proximale enroulée (176a) du ressort à force constante (176) tirant le prolongateur d'agrafes (174) de manière distale sur un enroulement de l'extrémité proximale enroulée (176a) du ressort à force constante (176) suite à un chargement d'une agrafe la plus distale (C_{d}) de la pluralité d'agrafes (C) dans la paire de mâchoires (120) de l'applicateur d'agrafes chirurgicales (100).
